(19) **Europäisches Patentamt**
**European Patent Office**
**Office européen des brevets**

(11) **EP 1 332 231 B1**

(12) **EUROPEAN PATENT SPECIFICATION**

(45) Date of publication and mention
of the grant of the patent:
**13.08.2008 Bulletin 2008/33**

(51) Int Cl.:
*C12Q 1/70* [(2006.01)] *C12Q 1/68* [(2006.01)]
*A61K 31/44* [(2006.01)] *A61K 31/505* [(2006.01)]
*A61P 31/18* [(2006.01)] *G06F 19/00* [(2006.01)]

(21) Application number: **01933853.2**

(22) Date of filing: **18.04.2001**

(86) International application number:
**PCT/EP2001/004445**

(87) International publication number:
**WO 2001/079540 (25.10.2001 Gazette 2001/43)**

(54) **METHODS FOR MEASURING DRUG RESISTANCE**

METHODE ZUR BESTIMMUNG DER RESISTENZ GEGEN MEDIKAMENTE

PROCEDES DE MESURE DE LA RESISTANCE AUX MEDICAMENTS

(84) Designated Contracting States:
**AT BE CH CY DE DK ES FI FR GB GR IE IT LI LU MC NL PT SE TR**

(30) Priority: **18.04.2000 US 197606 P**
**22.06.2000 US 213219 P**

(43) Date of publication of application:
**06.08.2003 Bulletin 2003/32**

(73) Proprietor: **VIRCO BVBA**
**2800 Mechelen (BE)**

(72) Inventors:
• **LARDER, Brendan**
**Cambridge,**
**Cambridgeshire CB3 7HQ (GB)**
• **BLOOR, Stuart**
**Biggleswade,**
**Bedfordshire SG18 8SR (GB)**
• **HERTOGS, Kurt**
**B-2018 Antwerpen (BE)**
• **DEHERTOGH, Pascale, Alfons, Rosa**
**B-2870 Puurs (BE)**
• **MORTIER, Rudy, Jean, Marc**
**B-9270 Laarne (BE)**

(74) Representative: **Daelemans, Frank F.R. et al**
**Tibotec-Virco Comm. VA**
**J&J Patent Law Department**
**Generaal De Wittelaan L 11B 3**
**2800 Mechelen (BE)**

(56) References cited:
**WO-A-01/16860**      **WO-A-99/67427**

• **BEROUD CHRISTOPHE ET AL: "UMD (Universal Mutation Database): A generic software to build and analyze locus-specific databases." HUMAN MUTATION, vol. 15, no. 1, January 2000 (2000-01), pages 86-94, XP002217108 ISSN: 1059-7794**
• **LARDER B ET AL: "A complete survey in over 1,500 clinical HIV-1 isolates of phenotypic and genotypic protease resistance profiles and their relation to therapy history" AIDS, vol. 12, no. suppl. 4, November 1998 (1998-11), page S11 XP000952507**
• **HERTOGS K ET AL: "Comprehensive HIV drug resistance monitoring using rapid, high-throughput phenotypic and genotypic assays with correlative data analysis" AIDS, vol. 12, no. suppl. 4, November 1998 (1998-11), page S11 XP000952505**
• **DATABASE BIOSIS [Online] BIOSCIENCES INFORMATION SERVICE, PHILADELPHIA, PA, US; 1997 PAUWELS R ET AL: "Correlation between genotypic and phenotypic resistance data in AVANTI." Database accession no. PREV199800113773 XP002217112 & ABSTRACTS OF THE INTERSCIENCE CONFERENCE ON ANTIMICROBIAL AGENTS AND, vol. 37, 1997, pages 263-264, 37th Interscience Conference on Antimicrobial Agents and Chemotherapy;Toronto, Ontario, Canada; September 28-October 1, 1997, 1997**

**(Cont. next page)**

• DATABASE BIOSIS [Online] BIOSCIENCES INFORMATION SERVICE, PHILADELPHIA, PA, US; December 1999 (1999-12) DE LEON M PONZ ET AL: "Clinical features and genotype-phenotype correlations in 41 Italian families with Adenomatosis Coli." Database accession no. PREV200000106882 XP002217113 & ITALIAN JOURNAL OF GASTROENTEROLOGY AND HEPATOLOGY, vol. 31, no. 9, December 1999 (1999-12), pages 850-860, ISSN: 1125-8055

• JEANPIERRE CECILE ET AL: "Software and database for the analysis of mutations in the human WT1 gene." NUCLEIC ACIDS RESEARCH, vol. 26, no. 1, 1 January 1998 (1998-01-01), pages 271-274, XP002217110 ISSN: 0305-1048

• LAURENT-PUIG PIERRE ET AL: "APC gene: Database of germline and somatic mutations in human tumors and cell lines." NUCLEIC ACIDS RESEARCH, vol. 26, no. 1, 1 January 1998 (1998-01-01), pages 269-270, XP002217111 ISSN: 0305-1048

**Description**

**FIELD OF THE INVENTION**

The present invention concerns methods and systems for predicting the resistance of a disease to a therapeutic agent. More specifically, the invention provides methods for predicting drug resistance by correlating genotypic information with phenotypic profiles. The invention further relates to methods and systems for designing, optimizing and assessing the efficiency of a therapeutic regimen based upon the genotype of the disease affecting the patient.

**BACKGROUND TO THE INVENTION**

Techniques to determine the resistance of a pathogen or malignant cell to a therapeutic agent are becoming increasingly important. For example, despite the great advantages of existing treatments against viral infections such as HIV infection, cancer and bacterial infections, many patients experience treatment failure or reduced efficacy over time. In many instances this Is due to the pathogen, malignant cell, bacteria, virus or other disease state mutating and/or developing a resistance to the treatment.

For example, all the drugs in the HIV field were discovered and developed over a period of 15 years, starting with AZT. By the beginning of the year 2000, 15 different anti-HIV-1 agents had been approved by the FDA. Initially, and due to a lack of alternative drugs, these agents were administered alone, as monotherapy. Though a temporary antiviral effect was observed, all the compounds lost their effectiveness over time. In 1989, Larder et al. published a paper In Science, 246, 1155-8, that identified a number of mutations that caused HIV-1 resistance to AZT. Since then, research has demonstrated that one of the main reasons behind treatment failure for all the antiviral drugs is the development of resistance of the virus to the drug.

Drug resistance and drug resistant mutations develop because retroviruses such as HIV have no proofreading mechanism when synthesizing new nucleic acid strands. This allows for the continuous generation of a number of genetic variants in a replicating viral population. More importantly, the genetic changes may alter the configuration of the reverse transcriptase (RT) and protease (PR) molecules In such a way that they are no longer susceptible to inhibition by compounds developed to target them. If antiretroviral therapy is ongoing and if viral replication is not completely suppressed, the selection of genetic variants is inevitable and the viral population becomes resistant to the drug.

In the face of monotherapy failure and encouraged by a number of clinical trials, in the early-mid 1990's treatment strategy turned to combination therapy, *i.e.*, administration of mixtures of antiviral drugs. At the time there were still only one class of drugs available - the nucleoside analogue reverse transcriptase inhibitors (NRTIs). As a result, the standard of care became two nucleosides, typically AZT+ddl, or AZT+ddC.

Dual combination therapy provided increased control of viral replication, made it more difficult for the virus to develop resistant strains or mutations and, as a result, provided extended clinical benefit to patients.

In 1995, another milestone was reached with the approval of the first of the protease inhibitors (Pls). These inhibitors showed greater potency than the nucleosides, but again were prone to resistance when used alone. Their combination with two nucleoside analogues, however, seemed to provide the control over the virus that everyone had been looking for. Triple combination therapy using two nucleosides (most commonly AZT+3TC) plus a protease inhibitor (typically indinavir) still remains the most common standard of care in developed countries.

These highly active combinations have had an enormous effect on the quality of life and on the survival of patients. This has resulted in fewer hospitalizations and reintegration of the patients in society. In a considerable number of patients, the viral load has been reduced to below the detection limit for prolonged periods.

In recent years, however, it has become clear that even patients being treated with triple therapy including a protease inhibitor often eventually experience treatment failure. Data suggests that up to one half of patients on combination therapy do not achieve or do not maintain suppression of virus replication. In some cases, it may be that even state-of-the-art triple therapy is insufficient to halt viral replication. As a result, drug resistant strains of the virus develop.

Another factor contributing to the difficulty to maintain suppression of virus replication has been the sheer burden of taking up to 20 pills each day, at set times, with or without food, day after day. It is simply unrealistic to expect people to adhere to such stringent and demanding regimens indefinitely. But if patients do not adhere, the price can be high. A dip in the blood levels of any of the medications gives the virus an opportunity to replicate and develop drug resistant strains. As such, during the course of infection, drug resistant viral strains can emerge very rapidly particularly for retroviral infections such as HIV-1. In addition, not all HIV-1 infections originate with a wild type, drug sensitive strain from which drug resistance will emerge. With the increase in prevalence of drug resistant strains comes the increase in infections that actually begin with drug resistant strains. Infections with pre-existing drug resistance immediately reduce the drug options for drug treatment and emphasize the importance of drug resistance information to optimize initial therapy for these patients.

Moreover, as the number of available antiretroviral agents has increased, so has the number of possible drug combinations and combination therapies. However, it is not easy for the physician to establish the optimal combination for an individual. Previously, the only treatment guidelines that have been in widespread use have been based on viral load and, where available, the patient's treatment history. The physician's objective is to keep the viral load as low as possible. An increase in viral load is a warning that control of viral replication is being lost and that a change in therapy is required. Viral load, however, provides no information or guidance regarding which drugs should be used.

Knowledge of the resistance patterns of different inhibitors and the patient's treatment history can help. Resistance emergence is highly predictive of treatment failure. In fact, while there are a variety of factors that can contribute to the failure of drug therapy, HIV-1 drug resistance is almost always involved. However, the interactions between different viral mutations related to different inhibitors is so complex that selecting the optimal treatment combination with only a treatment history to go on is far from ideal. Drugs can be ruled out unnecessarily and ineffective drugs can be introduced. Even if the virus is resistant to just one of three drugs in a treatment regimen, this can allow low-level viral replication to take place and viral strains resistant to the other two drugs to develop.

It is clear that although there are many drugs available for use in combination therapy, the choices can quickly be exhausted and the patient can rapidly experience clinical progression or deterioration if the wrong treatment decisions are made. The key to tailored, individualized therapy lies in the effective profiling of the individual patient's virus population in terms of sensitivity or resistance to the available drugs. This will mean the advent of truly individualized therapy.

The aim of resistance monitoring is to provide the necessary information to enable the physician to prescribe the most optimal drug combination for the individual patient. At present, there are two distinct approaches to measuring resistance:

The first approach involves phenotyping, which directly measures the actual sensitivity of a patient's pathogen or malignant cell to particular therapeutic agents. For example, HIV-1 phenotype testing directly measures HIV-1 drug resistance, detected as the ability of HIV-1, taken from a patient, to grow in the presence of a drug, in the laboratory. The phenotype is measured, for example expressed an $IC_{50}$ or as a fold resistance for a particular drug, which is defined as the concentration of drug required to kill half of the virions in a sample. This is compared to the $IC_{50}$ for the drug using wild type virus. The phenotype is usually described or can be expressed in terms of the fold increase in $IC_{50}$ for each of the drugs.

There are three main types of methodology for phenotyping. One such type is the plaque reduction assay. A drawback of this method is that it does not detect NSI strains. Another method of phenotyping includes PBMC p24 growth inhibition assays (Japour, A.J., Mayers, T.L., Johnson, V.A., Kuritzkes, D.R., Beckett, L.A., Arduino, J.-M., Lane, J., Black, R.J., Reichelderfer, P.S., D'Aquila, R.T., Crumpacker, C.S., The RV-43 Study Group & The ACTG Virology Committee Resistance Working Group. 1993. Antimicrob. Agents Chemother. 37, 1095-1101,

A problem with this technique is that virus culture from PBMCs is very slow and labor-intensive. In addition, it lacks the precision of other techniques and because it relies on primary human cells for virus growth, assay automation and high throughput is virtually impossible. Yet another method is the recombinant virus assay (Kellam, P. & Larder, B.A. 1994. Antimicrob. Agents Chemother. 38, 23-30,

). The recombinant method has advantages over the previously mentioned assays in that it reduces the amount of selection that takes place during growth of the virus in the laboratory, it is faster, more reproducible, amendable to automation and high throughput, and all available drugs can be tested in one assay.

The second approach to measuring resistance involves genotyping tests that detect specific genetic changes (mutations) in the viral genome which lead to amino acid changes in at least one of the viral proteins, known or suspected to be associated with resistance.

There are a number of techniques for conducting genotyping, such as hybridization-based point mutation assays and DNA sequencing. Common point mutation assays include Primer-specific PCR (Larder BA, Kellam P & Kemp, SD 1991. AIDS 5: 137-144), differential hybridization (Eastman, P.S., Urdea, M., Besemer, D., Stempien, M. & Kolberg, J. 1995. J. Acquir. Immune Defic. Syndr. Human Retrovirol. 9, 264-273, ), Line Probe Assay (*LiPA*™, *Innogenetics)* (Stuyver, L., Wyseur, A., Rombout, A., Louwagie, J., Scarcez, T., Verhofstede, C., Rimland, D., Schinazi, R. F. & Rossau, R. 1997. Antimicrob. Agents Chemotherap. 41, 284-291 .), and gene chip sequencing (Affymetrix) (D'Aquila, R.T. 1995. Clin. Diagnost. Virol. 3, 299-316 . Point mutation assays can only provide a small select part of the resistance picture. DNA sequencing, however, provides information on the nucleotides in the region of the genome sequenced. This means that changes in the genome can be detected. However, at present, it remains difficult to interpret the results of a genotypic test to provide meaningful conclusions about therapeutic agent resistance. The advantage of phenotyping over genotyping Is that phenotyping is a direct measure of any change In sensitivity resulting from all the mutations that have occurred, and any interactions between them. As such, it is the gold standard of resistance testing. Disadvantages of phenotyping are that it Is complex, lengthy to perform, (usually 4 weeks) and, therefore, more expensive than genotyping. Thus, phenotyping is not a practical way of designing patient therapy.

In WO 99/67427 a list of mutations is described known to influence the sensitivity of HIV to drug therapy. The method uses nucleic acid amplification and established (using a phenotypic susceptibility assay) that mutations at several codons are correlated with a decrease in specific drug susceptibility.

In AIDS, vol 12, suppl 4, Nov 1998, page S11 is disclosed a survey of HIV isolates of phenotypic and genotypic protease inhibitor resistance profiles utilizing a test for phenotypic susceptibility to antiretroviral drugs, the so-called Antivirogram method ® based on plasma RNA derived recombinant virus.

In Nucleic Acids Research pages 271-274 of 1998, Jeanpierre et al, disclosed a cancer related genotype-phenotype correlation analyses using a computerized database of germline (70 entries) and somatic (28 entries) mutations of the WT1 gene reported in the literature.

The importance of the speed by which a physician can be informed of the patient's resistance profile can be demonstrated by the following hypothetical but realistic example, which highlights the need to reduce complexity and improve performance time of assessing resistance. Suppose first-line triple combination therapy reduces the viral load to undetectable limits for a period of time. The viral load then begins to increase as a result of the development of resistance. Without resistance information, the physician can make a judgement based on the patient's treatment history, and change one or more of the drugs. As a result viral load is, again, reduced but the new treatment regimen is suboptimal so viral replication continues under selection pressure from the drugs and resistance rapidly develops once more. Consequently, control of viral replication is lost and several of the 15 drugs available have been 'used up'.

Although genotyping tests can be performed more rapidly, a problem with genotyping is that there are now over 100 individual mutations with evidence of an effect on susceptibility to HIV-1 drugs and new ones are constantly being discoverer, in parallel with the development of new drugs and treatment strategies. The relationship between these point mutations, deletions and insertions and the actual susceptibility of the virus to drug therapy is extremely complex and interactive. An example of this complexity is the M184V mutation that confers resistance to 3TC but reverses AZT resistance. The 333D/E mutation, however, reverses this effect and can lead to dual AZT/3TC resistance.

Consequently, the interpretation of genotypic data is both highly complex and critically important. There have been a number of different approaches to this challenge of interpretation. For example, armed with the knowledge of the main resistance mutations associated with each drug and the patient's recent treatment history, a physician makes a decision as to the optimum treatment To assist physicians to make these judgements, various expert opinion panels have been convened and have published guidelines, e.g. the Resistance Collaborative Group. In addition, rules-based algorithms constitute another approach. This is essentially a formalized version of the above with tables giving the mutations which are associated with resistance to each of the drugs. These can be simple printed tables or the information can be used to develop a rules-based computer algorithm. However, given the large number of mutations that are involved in resistance to antiretroviral drugs and given the complex interactions between the mutations, the shortcoming of genotyping is the reliable interpretation and clinical application of the results. As more drugs become available and as more mutations are involved in the development of resistance, the 'manual' or rules-based interpretation of raw genotype data is rapidly becoming impossible due to an increase in complexity.

Therefore, the main challenge involved with genotyping is improving the interpretation of the results. The technology will identify some (i.e., point mutation assays) or all of the mutations (i.e., DNA sequencing) that have occurred but it then requires sophisticated interpretation to predict what the net effect of these mutations might be on the susceptibility of the virus population to the various therapeutic agents. A physician might then have to combine this information with all the other information relating to the patient and decide what all this means in terms of selecting drugs for the treatment of their individual patient.

It is therefore an aim of the present invention to provide methods for improving the interpretation of genotypic results.

It is a further aim of the invention to provide methods for determining (or predicting) a phenotype based on a genotype.

It is also a further aim of the invention to provide methods for predicting the resistance of a pathogen or a malignant cell to a therapy or a therapeutic agent.

It is also an aim of the invention to predict resistance of a patient to therapy.

It is also an aim of the invention to provide methods to assess the effectiveness or efficiency of a therapy or to optimize a patient's therapy.

## SUMMARY OF THE INVENTION

A solution to the problems set forth above involves new methods for measuring drug resistance by correlating genotypic information with phenotypic profiles.

In the present invention, the methods bring together the knowledge of both a genotypic and a phenotypic database, and determines a (virtual) phenotypic fold resistance value without actually having to do phenotypic testing. The genotypic database contains the mutations in the tested HIV viruses compared with the reference HIV virus (wild type). The phenotypic database contains phenotypic resistance values for the tested HIV viruses, with a fold resistance determination compared to the reference HIV virus (wild type). As described below, this analysis may be done by comparing the sequence of the HIV virus sequence under test, e.g. from a patient sample, against the stored sequences and by selecting "similar sequences". Phenotypic data is then gathered for those "similar sequences" and the mean or median fold resistance may be calculated from the selected phenotypic values. This value is called "Virtual Fold Resistance", which leads to the "Virtual Phenotype."

## DETAILED DESCRIPTION OF THE INVENTION

According to a first embodiment the present invention relates to a method for determining or predicting a virtual phenotype of a human immunodeficiency virus for example in a biological sample, comprising:

a) obtaining a genetic sequence from said virus,
b) identifying at least one mutation pattern in said genetic sequence wherein said genetic sequence comprises at least one mutation, and wherein said at least one mutation or mutation pattern is to be associated with resistance to at least one therapy or therapeutic agent,
c) searching a genotype database for genotype entries with a similar mutation pattern to at least one of the mutation patterns identified in the genetic sequence in b),
d) correlating each genotype entry with a similar mutation pattern with a phenotype in a phenotype database , and
e) calculating the virtual fold resistance of said virus from all the database phenotypes identified.

The same methodology of the above described method can be used for instance for evaluating currently applied therapies or for predicting resistance of a patient to a therapy.
Therefore, according to another embodiment, the present invention relates to a method for assessing the effectiveness of a patient's therapy or for monitoring a patients therapy comprising:

a) providing a biological sample from a patient.
b) obtaining a genetic sequence from a human immunodeficiency virus in said biological sample.
c) Identifying at least one mutation pattern in said genetic sequence wherein said genetic sequence comprises at least one mutation, wherein said at least one mutation is associated with resistance to at least one therapy currently being administered to the patient,
d) searching a genotype database for genotype entries with a similar mutation pattern to at least one of the mutation patterns identified in the genetic sequence in c),
e) correlating said genotype entries with a similar mutation pattern with a phenotype in a phenotype database,
f) calculating the virtual fold resistance of said virus from all the database phenotypes identified,
g) obtaining a series of phenotypes by repeating steps b) through e) for each therapy currently being administered to the patient, and,
h) evaluating the effectiveness of the patient's therapy from the series of phenotypes.

Also, the invention relates to a method for optimizing therapy for a patient, comprising:

a) providing a biological sample from a patient.
b) obtaining a genetic sequence from a human immunodeficiency virus in said biological sample,
c) Identifying at least one mutation pattern in said genetic sequence wherein said genetic sequence comprises at least one mutation, and wherein said at least one mutation is associated with resistance to at least one therapy,
d) searching a genotype database for genotype entries with a similar mutation pattern to at least one of the mutation pattern identified in the genetic sequence in c),
e) correlating said genotype entries with a similar mutation pattern with a phenotype in a phenotype database,
f) calculating the virtual fold resistance of said virus from all the database phenotypes identified,
g) obtaining a series of phenotypes by repeating steps b) through e) for a group of therapies, and,
h) optimizing therapy for the patient from the series of phenotypes.

While described in the examples with respect to viruses, particularly HIV, the present invention has broad applicability to any disease state where it is desired to correlate genotypic information with phenotypic profiles. One skilled in the art could readily take the following discussion of the invention with the HIV virus and through the exercise of routine skill apply this invention to other diseases (such as other viral infections, malignant cells, cancer, bacterial infections, other pathogens, and the like) to correlate genotypic information to predict phenotypic response, assess drug resistance, and eventually develop a treatment regime of drugs for a particular patient. One skilled in the art will also know that many virus species comprise many strains for instance HIV comprise apart from HIV-1 also HIV-2 and both groups are further divided into groups e.g. but not limited to group Q or M for HIV-1.

Therefore, according to another embodiment, the present invention relates to a method for predicting resistance of a human immunodeficiency virus therapy comprising:

a) providing a biological sample from a patient containing a human immunodeficiency virus,

b) obtaining, a genetic sequence from said virus,

c) Identifying at least one mutation pattern in said genetic sequence wherein said genetic sequence comprises at least one mutation, and wherein said at least one mutation is associated with resistance to at least one therapy,

d) searching a genotype database for genotype entries with a similar mutation pattern to the mutation pattern identified in the genetic sequence In c),

e) correlating said genotype entries with a similar mutation pattern with a phenotype in a phenotype database,

f) obtaining a series of phenotypes by repeating steps b) through e) for a group of therapies, and,

g) predicting resistance of the patient to therapy from the series of phenotypes.

The above methods should be interpreter as diagnostic methods, therefore, the invention also provides diagnostic kits for performing each of the method of the invention.

It should be understood that the principles and methods provided by this application are governed to provide the treating physician a means to optimize or to select the therapy which will be most successful. The principle is of particular relevance for the treatment (or monitoring of therapy) of viral infections. These diseases states are subject to complex and continuously varying therapy regimens and therefore the patient under treatment needs to undergo frequent therapy monitoring in order to follow the drug effect or in order to optimize or select the optimal patient management.

The methods of the present invention determine a phenotype without actually having to do phenotypic testing. Within this meaning, the term "determining" is interchangeable with "predicting" or "diagnosing".

A "patient" may be any organism, particularly a human or other mammal, suffering from a disease or in need or desire of treatment for a disease. A patient includes any mammal, including farm animals or pets, and includes humans of any age or state of development

A "biological sample" may be any material obtained in a direct or indirect way from a patient comprising a disease producing or a disease causing agent. Said disease producing agent is able to be sequenced. In this respect the terms biological sample and disease producing agents and disease causing agents are interchangeable in the invention. A biological sample may be obtained from, for example, saliva, semen, breast milk, blood, plasma, feces, urine, tissue samples, mucous samples, cells in cell culture, cells which may be further cultured, etc. Biological samples also include biopsy samples. In one embodiment, for a patient infected with HIV, any biological sample containing virus may be used.

In one embodiment, for a patient infected with a virus, any biological sample containing virus may be used in any of the methods of the invention. Preferably said virus is a retrovirus. Preferably the biological sample contains a virus chosen from HIV, HCV-(Hepatitis C Virus) and HBV (Hepatitis B virus).

"HIV" is the human immunodeficiency virus, which is a retrovirus. "Retrovirus" is any RNA virus that utilizes reverse transcriptase during its life cycle. "HCV" is the human hepatitis virus, which is an RNA virus. "HBV" is the human hepatitis B virus, which is a DNA virus, but which shares some characteristics of retroviruses, in that is also displays a reverse transcriptase activity by which genomic RNA is translated to DNA within the virus.

According to yet another preferred embodiment, the biological sample in any of the methods may contain cells, tissue cells,

In one embodiment, a target nucleic acid or protein is present from which a genetic sequence or protein sequence can be derived is present in the biological sample.

A "genetic sequence" is any sequence containing at least one nucleotide. A nucleotide, for example, may be represented by the letters A, C, T or G. A nucleotide, for example, may be represented by the letters A, C, T or G. A combination of nucleotides, may be represented, for example, by other letters such as R, Y, M, etc. The amino acids are represented by their own code. An overview of the abbreviations used for nucleic acids and amino acids can be found in Alberts, B., Bay, D., Lewis, J., Raff, M., Roberts, K., Watson, J. The Molecular Biology of the Cell, Garland publishing, New York, 1994.

Genetic sequences as used herein may refer to the complete sequence of a disease producing agent or at least one segment of the sequence of a disease producing agent. The sequence of a particular target protein can be obtained by either sequencing the nucleic acid coding for the target protein or by sequencing the protein itself. Protein sequencing can be obtained for example but not limited to classical Edman degradation chemistry ("Sequence determination" Edman P. Mol. Biol. Biochem. Biophys. 1970, 8, 211-255.). This chemistry can also be fully automated.

Novel techniques including mass spectroscopy also enable the analysis of the sequence of a protein under investigation ("Mass spectroscopy from genomes to proteomics" Yates J. Trends in genetics 2000,16, 5-8) Alternatively the sequence of a target protein can be obtained using classical nucleic sequencing protocols e.g. extension chain termination protocols (Sanger technique) (("DNA sequencing with chain terminating inhibitors" Sanger F., Nichler., Coulson A. Proc. Nat. Acad. Sci. 1977, 74, 5463-5467.) or chain cleavage protocols. Particular sequencing methodologies were developed by e:g. Visible Genetics. It should be understood that novel approaches have been developed for unravelling the sequence of a target nucleic acid including but not limited to mass spectrometry, MALDI-TOF (matrix assisted leaser desorption ionization time of flight spectroscopy) ("Differential sequencing with mass spectroscopy" Graber J, Smith C., Cantor C. Genet. Anal. 1999, 14, 215-219.) chip analysis (hybridization based techniques) (Multiplexed biochemical assays with biological chips. Fodor S P; Rava R P; Huang X C; Pease A C; Holmes C P; Adams C L Nature 1993, 364, 555-6.) It should be appreciated that nucleic acid sequencing covers both DNA and RNA sequencing.

The term "codon" whenever used in the present invention relates to the position of the amino acid present at that specific location of the gene investigated. E.g. a mutation at codon 90 of the protease gene refers to the an altered amino acid at position 90 in the protein chain as compared to the wild type gene.

The nucleic acid can be present in the biological sample In a free and/or soluble form, or can be encapsulated by proteins, such as in viruses. In preferred embodiments of the invention, the nucleic acid may be present in a cell, such as a tissue cell,

The term "pathogen" may relate to any bacterium, virus, fungus or any other microorganism or multicellular organism which causes a disease state in another organism. Said other organism preferably is a mammal, most preferably a human mammal. However said other organism can also be a plant or a plant cell wherein said pathogen causes a disease state.

The term "phenotype" may include any observable property of an organism or disease producing agent that is produced by the genotype in conjunction with the environment.

In one embodiment, phenotype refers to resistance of a disease producing agent to at least one therapy. Therefore, the methods of the invention determine a phenotype of a disease producing agent towards at least one therapy or therapeutic agent.

The expression "virtual phenotype" relates to a phenotype of a sample that is obtained through the determination of the genotype of said sample, said genotype is used for correlation in a database to search for matching genotypes for which a corresponding phenotype is known. From this collection of phenotypes the phenotype of the sample is calculated.

The methods of the invention can be repeated for each possible therapy or therapeutic agent known or suspected to be associated with resistance, or towards which a resistance can be expected to appear. As such, according to another embodiment of the invention, the phenotype of a biological sample can be presented as a list of phenotypes against or in respect of individual therapies or individual therapeutic agents. This is further illustrated in the examples section.

The expression "phenotypic resistance" comprises resistance of a cell, virus, or virally infected cell to a tested therapy, therapeutic agent or drug.

The term "resistance" as used herein, pertains to the capacity of resistance, sensitivity, susceptibility, or effectiveness of a therapy against a disease.

The term "therapy" includes but is not limited to a drug, pharmaceutical, bactericide, fungicide, antibiotic, or anticancer, antiviral, anti-bacterial anti-fungal, anti-parasitical or any other compound or composition that can be used in therapy or therapeutic treatment. Therapy also includes treatment, such as gene therapy or radiation therapy, useful for the treatment or amelioration of a disease in a patient. Therapy, as used herein, also includes combination therapies.

The present invention can also be applied to determine the phenotype of normal (tissue) cells or non-malignant cells to investigate their behavior towards a particular therapy or therapeutic agent.

The term "mutation" as used herein, encompasses both genetic and epigenetic mutations of the genetic sequence of the disease causing agent. A genetic change includes, but is not limited to, (i) base substitutions: single nucleotide polymorphisms, transitions, transversions, substitutions and (ii) frame shift mutations: insertions, repeats and deletions. Epigenetic changes include, but are not limited to, alterations of nucleic acids, *e.g.*, methylation of nucleic acids. For instance (changes in) methylation of cytosine residues in the whole or only part of the genetic sequence. In the present invention, mutations may also be considered at the level of the amino acid sequence, and comprise, but are not limited to, substitutions, deletions or insertions of amino acids.

The "control sequence" or "wild type" is the reference sequence from which the existence of mutations is based. For example, a control sequence for HIV is HXB2. This viral genome comprises 9718 bp and has an accession number in Genbank at NCBI M38432 or K03455 (gi number: 327742).

Reference or wild type sequences for use in the invention in the field of specific diseases, infections or diseases caused by specific pathogens can be easily obtained from publicly available databases. For example, the influence of mutations on the etiology of cancer can be exemplified by the mutations influencing the effect of the tumor suppressor gene such as p53, TGF-beta, NF-1, WT-1, and Rb. Also, mutations present in oncogenes such as Ras, c-myc, c-raf, neu, and IL-2, and repair genes, *e.g.*, methylguanosyl and methyltransferase can cause changes in the phenotype and/or drug effect.

In another embodiment, a mutation that is a methylation of nucleic acids may occur at the 5-position of cytosine within the CpG-dinucleotide. In general the CpG dinucleotide is greatly under-represented throughout the mammalian genome, but it can be found at close to its expected frequency in small genomic areas of about one kilobase, called CpG islands. Although the CpG islands account for only about 1% of the complete genome and for 15 % of the total genomic CpG sites, these regions contain approximately 50% of the unmethylated CpG dinucleotides. Methylation, may for example, impact disease states, such as Fragile X and Rett syndrome, and also on drug profiling. See for example, Robertson et al., Nature Reviews, 2000 vol 1, p. 11-19, and Esteller M. et al. New England Journal of Medicine, 2000, Vol 343:19, p. 1350-1354

The expression "at least one mutation that correlates to resistance to at least one therapy" includes, but is not limited to, mutations and combination of mutations in a genetic sequence that influence sensitivity of a disease causing agent to a therapy.

The at least one mutation may influence sensitivity to a specific therapy, *e.g.*, a drug, or a group of therapies. The at least one mutation may, for example, increase and/or decrease resistance of a disease producing agent to a therapy. The at least one mutation, may also, for example, enhance and/or decrease the influence of other mutations present in a genetic sequence that effect sensitivity of a disease producing agent to a therapy.

In one embodiment, the at least one mutation that correlates to resistance to at least one therapy includes mutations or combinations of mutations that are known or suspected to influence the sensitivity to a therapy. Lists of mutations known or suspected to influence the sensitivity of a disease producing agent to a therapy may be found, for example, in the scientific literature, patents, and patent applications, e.g. Schinazi, R.F., Larder, B.A. & Mellors, J.W. 1997. Int. Antiviral News. 5, 129-142 (1997); WO 00/78996; WO 99/67427; WO 99/61658; US 6,087,093; WO 00/73511; and U.S. Patent Application Serial No. 09/580,491, U.S. Patent Application Serial No. 09/589,167 and "Method and system for predicting therapeutic agent resistance and for defining the genetic basis of drug resistance using neural networks

Examples of mutations known or suspected to influence the sensitivity of a disease producing agent to a therapy may also be found on the internet at http://hiv-web.|an|.gov; http://hivdb.stanford.edu/hiv/; or http://www.viral-resistance.com. Additional examples of mutations present in the RT domain of HIV conferring resistance to a reverse transcriptase inhibitor include, but are not limited to, 69 C, 69 V, 69 T, 75A, 101I, 103T, 103N, 184T, 188H, 190E, 219 N, 219 Q, 221 Y, 221I, and 233V. Additional examples of mutations present in the PR domain of HIV conferring resistance to a reverse transcriptase inhibitor include, but are not limited to, 24M, 48A, and 53L. A mutation may effect resistance alone or in combination with other mutations. The specific therapy, for example an antiretroviral drug, for which a mutation may effect resistance may be determined by one of skill in the art, for example, using the a phenotypic resistance monitoring assay such as, the ANTIVIROGRAM ®.

There are different possibilities to represent mutations in sequences In the form of mutation patterns, some of which are explained in detail in the examples section.

The expression identifying a mutation patter" in a genetic sequence relates to the identification of mutations in a genetic sequence under test compared to a wild type sequence which lead to a change in nucleic acids or amino acids or which lead to altered expression of the genetic sequence or altered expression of the protein encoded by the genetic sequence or altered exp ression of the protein under control of said genetic sequence.

A "mutation pattern" comprises at least one mutation influencing sensitivity of at least one disease causing agent to at least one therapy. As such, a mutation pattern may consist only one single mutation. Alternatively a mutation pattern may consist of at least two, at least three, at least four or at least five mutations.

According to yet another embodiments a mutation pattern is a list or combination of mutations or a list of combinations of mutations that influence sensitivity of at least one disease causing agent to at least one therapy. A mutation pattern may be constructed, for example, by searching a genetic sequence for the occurrence of each mutation of a series of mutations. The existence of a mutation or the existence of one of a group of mutations may then be noted. The mutation pattern is constructed, for example, once a genetic sequence is searched for the occurrence of each mutation in the series. In one embodiment, a mutation pattern is constructed using a group of mutations that correlate to resistance to a therapy, thereby constructing a mutation pattern that is specific to a therapy. In a further embodiment, a mutation pattern is constructed by searching for mutations in a genetic sequence wherein the mutations are linked by at least one logical operator chosen from AND, OR, NOT, and NOR.

In one embodiment the invention relates to any of the methods described in the invention wherein the mutation pattern comprises at least two mutations known or suspected to be associated with resistance to at least one therapy.

Furthermore, the present invention also relates to the identification of "at least one mutation pattern" in a sequence: It should be clear from the following, that for each biological sample (or for each genetic sequence derivable from said biological sample) several (i.e. more than one) mutation patterns can be identified towards a single therapy or a single therapeutic agent.

In one embodiment of the invention, the sequence under test is aligned with the wild type sequence and the alignment or differences in the alignment are stored in a computer medium or a database. Alternatively, a mutation pattern can be obtained from the alignment, represented by the mutated amino acids and their positions in the polypeptide(s). It should be clear that the man skilled in the art knows different ways of representing and/or handling information from sequence alignments including the use of known computer programs and algorithms ("Bioinformatics: A practical guide to the analysis of genes and proteins" Eds. Baxevanis and Ouellette, 1998, John, Wiley and Sons, New York. Chapter 7 "Sequence alignment and database searching" G. Schuler, Chapter 8 Practical "Aspects of multiple sequence alignment" A. Baxevanis and Chapter 9 "Phylogenetic analysis" M. Hershkovitz and D. Leipe). A practical example of multiple sequence alignment is the construction of a phylogenetic tree. A phylogenetic tree visualizes the relationship between different sequences and can be used to predict future events and retrospectively to devise a common origin. This type of analysis can be used to predict a similar drug sensitivity for a sample but also can be used to unravel the origin of different patient sample (i.e. the origin of the viral strain).

According to preferred embodiments of any of the methods of the invention, the similar mutation pattern is identified by aligning the genetic sequence of a cell or a pathogen in the biological sample with the WT genetic sequence of said cell or pathogen.

According to another embodiment, "Discrete Clustering" is used to determine when sequences are "similar". "Similar" in this context does not mean "exactly" alike, since no single sequence matches another. Rather, "similar", in this context, means " having similar mutations", or "having mutations that have the same effect towards resistance against inhibitor drugs." To be able to define the patterns of mutations that were regarded as "having the same effect", a pattern database that is drug related may be built. The patterns of mutations referred to here are called "hot spots". The term "hot-spot" is herein be defined as a combination of mutations that confer resistance to a defined drug.

The hot spots describe mutations or clusters of mutations (generally combined by "OR" (I) or "AND" (&) logical operators) that are related to a certain inhibitor drug. A drug may have 1, 2, 3, 4 or more hot spots attached to it. Other logical operators may be "NOT", "NOR" etc. and the possibility to identify INSERTS and DELETIONS in the DNA sequence.

A simplified example of, for instance, a hot spots table used for testing resistance of HIV sequences towards different drugs can be represented as follows:

| Drug | # | Hot spot |
|---|---|---|
| A | 1 | (mutations \| mutationE) &(mutationF \| mutationG) |
|  | 2 | mutationH \| mutationI |
|  | 3 | mutationJ & mutationK |
|  | 4 | (mutationZ \| mutationX) & mutationV |
| B | 1 | mutationL |
|  | 2 | mutationM & mutationN |
|  | 3 | (mutationO & mutationP) \| mutationQ |
| C | 1 | mutationR |
|  | 2 | mutationS \| mutationT |

Subsequently, every HIV virus sequence that is tested is "profiled" by testing the sequence against all the available hot spots, for all the inhibitor drugs involved. This analysis produces a profile per drug for the sequence of interest.

In one embodiment, for every hot spot that matches, the sequence receives a "1"; for every non-matching hot spot, it gets a "0". For a given sequence under test, the result could be:

| Drug | Profile |
|---|---|
| A | 1010 hot spots 1 and 3 apply, hot spots 2 and 4 do not for drug A. |
| B | 001 hot spot 3 applies, hot spots 1 and 2 do not for drug B. |
| C | 10 hot spot 1 applies, hot spot 2 does not for drug C. |

As such, the expression "therapy profile" or "drug profile" relates to the presentation of a genetic sequence as explained above. The term "therapy or drug profile" is the combination of mutation patterns corresponding to resistance to a single therapy or drug.

In other words, a therapy profile can be given for each drug. In the example of drug A above, hot spots 1 and 3 relate to resistance to drug A and are assigned a value of 1.

In contrast, hot spots 2 and 4 do not and are assigned a value of 0, thus the profile "1010". This procedure can be seen as a form of clustering. However, since the elements of the cluster (0 and 1) are based an pre-defined sets (hot spots) this method is usually referred to as "discrete clustering."

The present invention thus relates to any of the methods of the invention wherein discrete clustering is used to identify similar sequences or wherein cluster searching is used to determine similar mutation patterns.

According to a preferred embodiment, the invention relates to a method of determining a virtual phenotype of a human immunodeficiency virus comprising:

a) obtaining a genetic sequence of said human immunodeficiency virus,
b) identifying at least one mutation in said genetic sequence wherein said mutation is comprised within at least one mutation pattern,
c) searching a genotype database for similar genotype entries comprising said mutation in said at least one mutation pattern,
d) correlating said genotype entries with a phenotype in a phenotype database, and
e) calculating the virtual fold resistance of said virus from all the database phenotypes identified.

According to other preferred embodiments, the invention relates to a method for assessing the efficiency of a patient's therapy or for evaluating or optimizing a therapy comprising obtaining a biological sample containing a human immunodeficiency virus from a patient, further comprising at least steps a) to e) of the above described method.

The invention further relates to the above described methods wherein the mutation pattern is associated with resistance to one therapy or drug. In the above methods, steps b) to e) can be repeated to obtain a series of phenotypes for a group of therapies or drugs.

The invention further relates to the above described methods wherein said mutation pattern comprises at least two mutations linked with a logical operator, further characterized in that the at least two mutation patterns are associated with resistance to one therapy.

The invention further relates to the above described methods wherein said mutation patterns are linked with a logical operator defining a therapy profile and wherein said therapy profile is represented by a sequence, said sequence is represented by a series of 1 and/or 0 wherein 1 represents the presence of a mutation pattern in the therapy profile and 0 the absence of a mutation pattern in the therapy profile.

It should be understood that the principles and methods as outlined in the application are very dynamic. The databases are frequently updated to incorporate new mutations which improve the accuracy of the determination. The number and the combinations of mutations present in the system are update on a regular basis (every 3 to 4 months). This is necessary in order to incorporate newly identified mutations or combinations which improve the performance of the system. By taking less mutation (or hot-spots) one will still be able to calculate the phenotype, however, from a statistical perspective the performance of the system will lower. In addition this regular update is required to anticipate the effect of drugs which are added to the list and which may have their own list of mutations causing resistance to that drug.

The person skilled in the art will be aware of those mutations or combinations of mutations influencing the drug efficacy. Information hereon can be found at the internet http://hiv-web.lanl.gov, http://hivdb.stanford.edu/hiv/ or http://www.viral-resistance.com. or in articles e.g. Schinazi, R.F., Larder, B.A. & Mellors, J.W. 1997. Int. Antiviral News. 5, 129-142 (1997). In addition lists of mutations are provided in several patent applications. (Means and methods for monitoring protease inhibitor antiretroviral therapy and guiding therapeutic decisions in the treatment of HIV/AIDS (WO 00/78996), Means and methods for monitoring nucleoside reverse transcriptase inhibitor antiretroviral therapy guiding therapeutic decisions in the treatment of HIV/AIDS (WO 99/67427) Means and methods for monitoring non-nucleoside reverse transcriptase inhibitor antiretroviral therapy (WO 99/61658), Method for detection of drug-induced mutations in the reverse transecriptase gene (US 6,087,093), New mutational profiles in HIV-1 reverse transcriptase correlated with phenotypic drug resistance (WO 00/73511) and New mutational profiles in HIV-1 reverse transcriptase correlated with phenotypic drug resistance (US Pat. Ser. N°: 09/580/491)

In the present invention, after determining the hot spots or the therapy for a sequence under test, a genotype database may be queried for sequences similar to the sequence under scrutiny. This query may be done using cluster searches.

The expression "genotype database" relates to diverse types of databases wherein sequence information is stored. According to one embodiment of the invention, the genotype database stores complete or partial nucleotide sequences. According to other embodiments of the invention the genotype database stores nucleotide sequences linked to their amino acid translations or stores nucleotide sequences linked to at least one list of particular mutations. These mutations are in respect of a reference sequence. Also these mutations can be at the nucleotide level or at the amino acid level. These lists can include all mutations in respect of a reference sequence or can contain a selection of mutations.

The information provided to the genotype database therefore can also be in the form a complete or partial nucleic acid sequence related with a biological sample or can be in the form of a list of particular mutations representing a particular nucleic acid sequence related with a biological sample. Therefore, the term "genotype entry" relates to any form in which information is provided to the genotype database.

For instance, according to the present invention, a preferred way of listing mutations in a genotype database is listing mutations which are known or suspected to be associated with resistance to a particular therapy or therapeutic agent. As such, each genotype entry in the genotype database can be linked to several lists of mutations occurring in the genetic sequence related to a biological sample, each of those lists representative for mutations which are known or suspected to be associated with resistance to a particular therapy or therapeutic agent towards which a resistance is known or can be expected to appear.

Regardless of the method used to select "similar sequences", once a selection of "similar sequences" is found, the application queries the phenotypic database for phenotypic data belonging to those sequences. The phenotype database may be constructed in such a way that in a database entry a genetic sequence (related to a biological sample) is linked to a phenotype. Alternatively, phenotypes in a phenotype database may be linked to other means of presenting nucleotide sequence information, for instance a mutation pattern or mutation profile for a therapy, therapeutic agent or drug. Alternatively relational genotype/phenotype databases may be used in any of the methods of the invention to correlate genotypic with phenotypic information. In one embodiment, this process is done for each therapy, therapeutic agent or drug, again using cluster searches. The query returns a selection of phenotypic results for every therapy, therapeutic agent or drug listed. A statistical analysis may be performed on the data to remove outliers and the virtual fold resistance may be calculated. For example, per drug, the mean of the log (fold resistance values) may be used to calculate the virtual fold resistance and the interpretation of these numbers will generate a Virtual Phenotype. The virtual phenotype (Fold Resistance value) may then further be used to classify the virus as Sensitive (S), Intermediate (I) or Resistant (R).

"Resistance" is determined using the protocols described in Antivirogram® assay (WO 97/27480). Resistance is determined with respect to a laboratory reference strain HIV LAI/IIIB. The difference in $IC_{50}$'s between the patient sample and the reference viral strain is determined as a quotient. This fold change in $IC_{50}$ is reported and indicative of the resistance profile of a certain drug. Based on the changes in $IC_{50}$, cut-off values have been established to distinguish a sample from being sensitive or resistant to a certain drug.

The expression "relational genotype/phenotype database" refers to a database that brings together the knowledge of both a genotypic and phenotypic database. The genotypic databases, for example, contains genetic sequence information regarding at least one tested disease producing agent. The genetic sequence information may vary from the entire sequence of a disease producing agent to a segment of the sequence of a disease producing agent, to a mutation pattern. For example, the genetic sequence of tested HIV viruses or the mutation pattern of tested HIV viruses.

The phenotypic database contains phenotypic resistance values for the at least one tested disease producing agent to at least one therapy. For example, the phenotypic resistance values of tested HIV viruses, with a fold resistance determination compared to the reference HIV virus (wild type).

In one embodiment, for example, the methods may use different genotype and phenotype databases. As a sample is run during the analysis, the identified sequence entries and their corresponding phenotypes are found and "transferred" to a "Call Center Database". This call center is a third database, where the pheno-genotype results are combined and used for the calculation of the virtual fold resistance and the generation of the report. This database is a relational database.

In one embodiment, in a relational gentoype/phenotype database, the data entries are combined to yield a "2D" representation for each sample: $(x_i, y_i)$ where $x_i$ represents the phenotypic result, $y_i$ the genotypic. In another embodiment, the data entries are combined to yield a "3D" representation for each sample: $(x_i, y_i, z_i)$ where $x_i$ represents the phenotypic result, $y_i$ the genotypic result, and $z_i$ other information regarding the sample, such as a sample number.

Therefore, the present invention also relates to any of the methods described wherein a relational genotype/phenotype database is used for correlating the at least one genotype entry with a similar mutation pattern with a phenotype in said database.

According to a preferred embodiment, the present invention provides a thorough and reliable interpretation of genotypic information.by interrogating the genotype part of a relational genotype/phenotype database for identical or similar patterns of mutations to that of the patient sample under study. Once the matches are found, the corresponding phenotypes are accessed and the phenotypic information, the changes in $IC_{50}$ to the various drugs, is pooled and averaged to produce a phenotypic profile.

This profile, in one embodiment of the invention, may be based on data from hundred or thousands of real phenotypes with the same patterns of mutations. In another embodiment, the RT-PR region of the HIV-1 genome of a patient sample is sequenced and the sequence is used in the methods of the invention to interpret the genotype information. The virtual phenotype may then be used to design a therapy, which may be one or more drugs. In a further embodiment, proprietary software may be used to interpret the genotype information according to the methods of the invention.

In one embodiment, a more accurate phenotype may be obtained by constructing a mutation pattern using mutations that have been validated. One of skill In the art will recognize that there are numerous methods of validating whether a mutation correlates to resistance to at least one therapy, including but not limited to phenotype experiments, such as the ANTIVIROGRAM (Virco, Belgium) and clinical studies. (WO 97/27480)

In another embodiment, the number and the combinations of mutations used to construct a mutation pattern would be updated on a regular basis. This may be done in order to incorporate newly identified mutations or combinations which may improve the performance of the system. In one embodiment, a phenotype may be calculated from at least one mutation used to construct a mutation pattern, however, from a statistical perspective a more accurate phenotype may result from a greater number of mutations.

According to a further embodiment, in any of the methods of the invention the phenotype of said biological sample can be expressed as a mean fold-change in resistance towards at least one therapy, wherein said mean fold-change resistance is calculated from the database phenotype(s) of the at least one genotype entry with a similar mutation pattern. Preferably, the phenotype of said biological sample towards the at least one therapy or therapeutic agent is expressed as an $IC_{50}$. The IC values are inhibitory concentrations, wherein the $IC_{50}$ represents the concentration of a defined drug yielding half of the signal output as compared to a blank run comprising no drugs.

The invention further relates to a method for generating a report wherein said report comprises the phenotype determined (or predicted) using any of the methods of the invention. Several examples of reports are illustrated in the examples section. The report may contain the phenotype of a biological sample against at least one therapy or therapeutic agent. Preferably the phenotype of a biological sample against several therapies or therapeutic agents are listed in said report.

The term "health care provider" is understood to include any professional person authorized or trained to treat or take patient data and/or samples. Such persona include but are not limited to physicians, doctors, clinicians, health care workers, nurses, technicians, laboratories, etc.

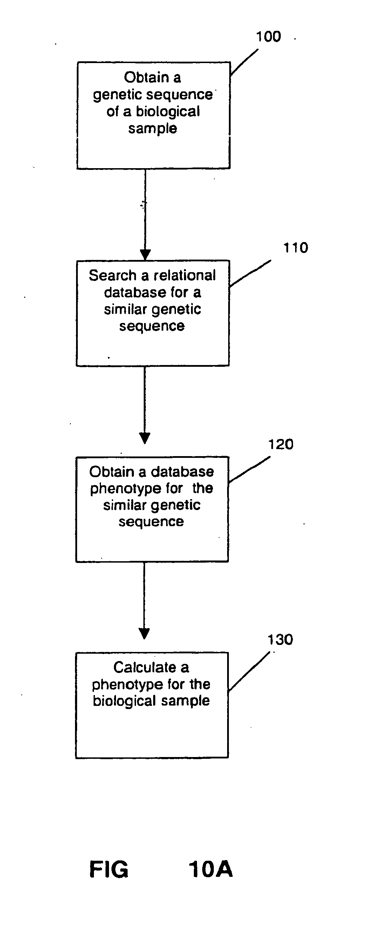

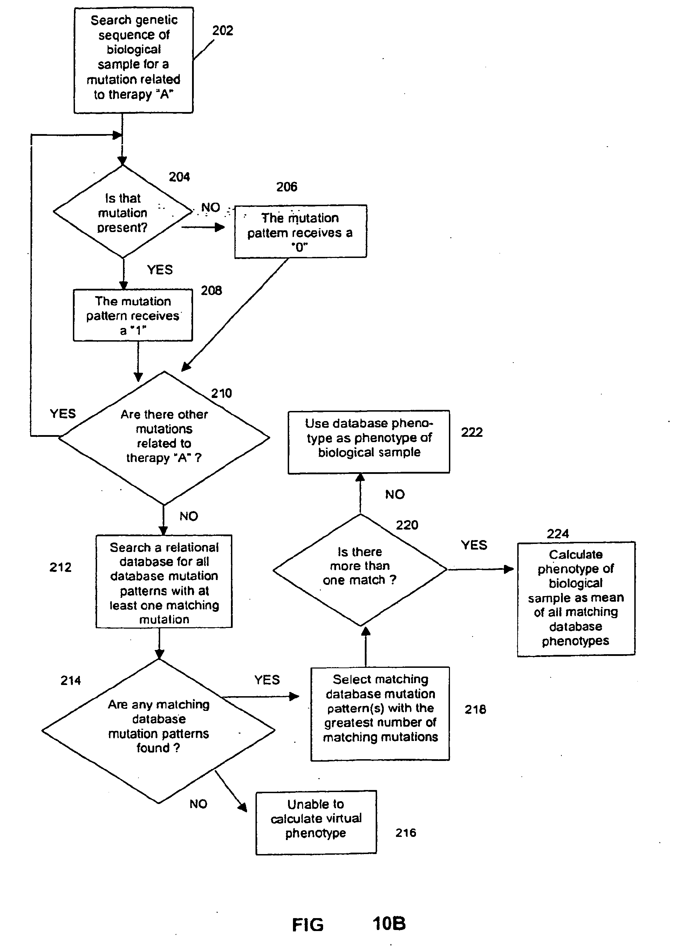

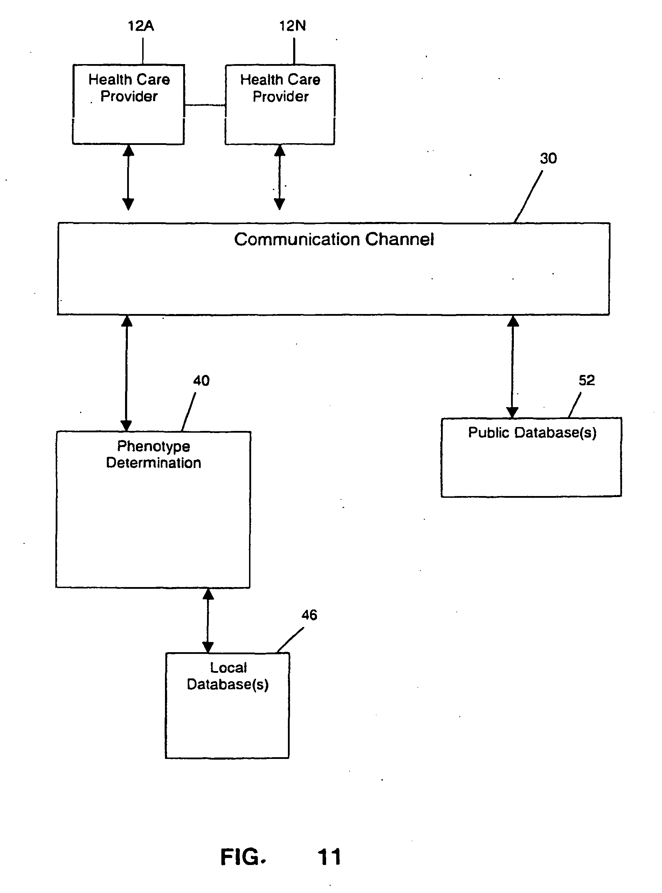

Figure 10 provides an exemplary flowchart for determining a virtual phenotype In one embodiment, the various steps and operations of Figure 10 may be performed by the phenotype determination system 40 in the system environment of Figure 11 to assess resistance of a patient to a therapy, or design or optimize a therapy for a patient, for example, with HIV.

As illustrated in Figure 10, in one embodiment the process starts with obtaining at least one genetic sequence of a patient (step 100). A genetic sequence may be obtained by a health care provider, laboratory, or any other entity. In one embodiment, the at least one genetic sequence, including genetic sequences taken at various times or a history of sequence of a patient may be stored in a database, such as local database 46 of phenotype determination system 40 (see Figure 11).

As part of computing a virtual phenotype, a mutation pattern of the genetic sequence may be determined (step 110) for at least one therapy. As part of this step, the phenotype determination system 40 may include data of mutations that correlate to resistance to at least one therapy. The mutation data may be accessed from local database 46 and/or public database(s) 52.

A relational genotype/phenotype database is then searched for at least one genetic sequence similar to the genetic sequence of the patient (step 120). All similar sequences are identified. This may be accomplished by searching for a mutation pattern similar to the mutation pattern determined in step 110 or, for example, by comparing the genetic sequence of the patient to sequences of the relational genotype/phenotype database using sequence alignment. The relational genotype/phenotype database may be accessed from a local database 46 and/or 46 and/or public database (s) 52.

As illustrated in Figure 10, a database phenotype is obtained for each similar genetic sequence identified from the relational genotype/phenotype database (step 130). A phenotype for the genetic sequence of the patient is then calculated from all of the database phenotypes identified (step 140).

The information may then be transmitted back to the health care provider or used in the determination of other information, such as assess resistance of a patient to a therapy, or to design or optimize a therapy for a patient. The resulting information may then be transmitted back to the health care provider. Figure 11 is an exemplary system environment in which the features and methods of the invention may be implemented (for example, the methods as shown in Figure 10). As illustrated in Figure 11, a communication channel 30 is provided for facilitating the transfer of data between various system components and entities. These components and entities may include, for example, one or more health care providers 12A-12N who interact with or treat patients (not shown), a phenotype determination system 40, and one or more public databases 52.

Communication channel 30 may be implemented through any single or combination of channels that allow communication between different people, computers, or locations. The communication channel may be any system that allows communication between the different entities illustrated in Figure 11.

Each of the health care providers 12A-12N, for example, collects biological samples for each patient or patients, and determines a genetic sequence or has a genetic sequence determined, wherein such data is submitted for analysis by phenotype determination system 40.

In one embodiment, the phenotype determination system 40 may be implemented through any suitable combination of hardware, software and/or firmware. For example, phenotype determination system 40 may be implemented through the use of a personal computer, a working station, a server or any other computing platform.

Software or programmed instructions may also be provided for controlling the operations of the computing platform, consistent with the principles of the invention.

As illustrated in Figure 11, phenotype determination system 40 may also include a local database 46 for storing patient data including genetic sequence data. Local database 46 may also store mutation data and/or relational genotype/phenotype data mutation data and/or relational genotype/phenotype data may be accessed from one or more public databases 52 by phenotype determination system 40.

Consistent with the methods of the present Invention, phenotype determination system 40 is configured to provide information regarding at least one of: phenotype, assessment of resistance of a patient to a therapy, and design or optimization of a therapy for patients treated by physicians 12A-12N. The information may be sent by system 40 to physicians 12A-12N in numerous formats (e.g., written report, electronic file, graphical display, etc.) and may be provided to physicians on fee basis or as a free or ancillary service.

It should be understood that the method as outlined in the Examples is apt to analyze the effect of genetic alterations, and the consequent protein changes, in the protease and reverse transcriptase gene of HIV. It should be appreciated that the method is equally well adaptable to analyze different genes or sets of genes present in HIV, or any other organism be it of viral, prokaryotic or eukaryotic origin, implicated in clinical diagnostics or in pharmacogenetics.

**DESCRIPTION OF FIGURES**

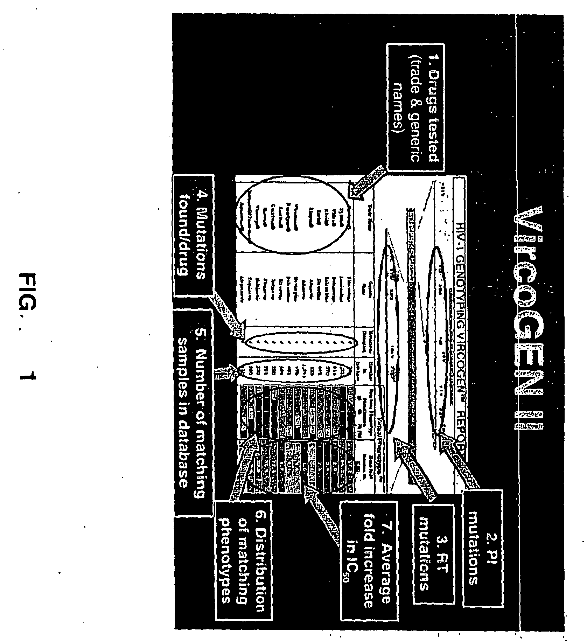

**Figure 1:** The report of Figure 1 provides the following information to aid the physician to interpret the genotypic data and develop a treatment regime:

1. The first two columns give the trade and generic names of the drugs.
2. The top of the chart has a graphic representation of the mutations in the protease region of the genome.
3. Below this is the same information for the reverse transcriptase region.
4. The third column simply indicates whether or not mutations affecting susceptibility for that particular drug were found.
5. The fourth column indicates the number of samples in the database that match the pattern of mutations in the sample virus, for each drug.
6. The fifth column has a color-coded representation of the range of phenotypic susceptibilities found in the database.
7. Finally the average $IC_{50}$ for all the matches in the database is presented for each drug.

**Figure 2:** A Prediction of a Phenotypic Report Using the Present Invention.

**Figure 3:** Predictive value of the present invention.

**Figure 4:** Section of the HIV genome covered by the Antivirogram® assay

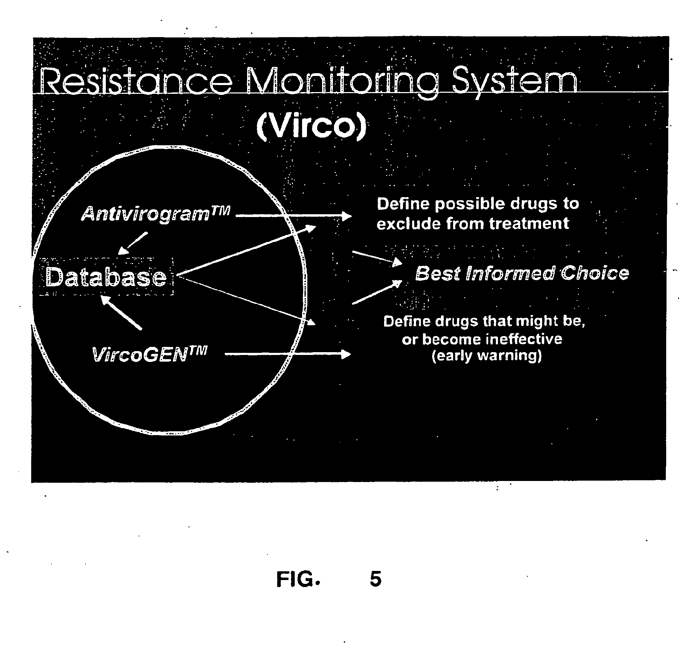

**Figure 5:** Schematic representation according to one embodiment of resistance monitoring.

**Figure 6:** is a schematic diagram of an exemplary pattern search The numbers indicated for each mutation (N) indicate the N observed in the database analysis illustrated in Table 1.

**Figure 7:** depicts the phenotypic search results for virus with different clusters of AZT resistance mutations. The graph shows the mean (o), standard error (■) and 95% confidence limits (⌐) for each cluster.

**Figure 8:** is a correlation between the actual and computer predicted virtual phenotype. A linear regression analysis is shown for four independent random data sets comprising 500 samples each.

**Figure 9 (a) & (b):** are a depiction of the odds ratios of failure to achieve a viral load reduction below 400 viral RNA copies/ml.

**Figure 10 (a) & (b):**

10 (a) is an exemplary flow chart for determining a phenotype, in accordance with the methods of the invention

10 (b) is an exemplary flow chart of one embodiment for performing step 110 to 130 of Figure 10 (a)

**Figure 11:** an exemplary representation of a system environment in which features and methods of the invention may be implemented.

**EXAMPLES**

**Example 1**

***Definition of a sequence.***

A sequence consists of a number of nucleotides. Nucleotides are represented by the letters A, C, T and G. A, C, T and G are the bases of a sequence. Other letters like R, Y, M etc. stand for a combination of two or more bases.

| Letter | MPX | Letter | MPX |
|--------|-----|--------|------|
| R | AG | H | ACT |
| Y | TC | B | GCT |
| M | AC | V | ACG |
| K | GT | D | AGT |
| S | CG | N | GATC |
| W | AT | | |

Groups of 3 nucleotides form a codon. These codons are translated to amino acids and then compared to a reference sequence in order to determine the mutations. A mutation is a difference between the reference sequence and the test sequence.

The raw nucleotide reference sequence looks like this (the example shows only the protease section which contains 99 amino acids or 297 nucleotides. The 'reverse transcriptase' section contains 400 amino acids or 1200 nucleotides.):

CCTCAGGTCACTCTTTGGCAACGACCCCTCGTCACAATAAAGATAGGGGG
GCAACTAAAGGAAGCTCTATTAGATACAGGAGCAGATGATACAGTATTAG
AAGAAATGAGTTTGCCAGGAAGATGGAAACCAAAAATGATAGGGGGAATT
GGAGGTTTTATCAAAGTAAGACAGTATGATCAGATACTCATAGAAATCTGT
GGACATAAAGCTATAGGTACAGTATTAGTAGGACCTACACCTGTCAACAT
AATTGGAAGAAATCTGTTGACTCAGATTGGTTGCACTTTAAATTTT

This is the protease section of the sequence under test:

CCTCAAATCACTCTTTGGCAACGACCCATCGTCACAATAAAAATAGGAGG
GCAACTAAGGGAAGCTCTATTAGACACAGGAGCAGATGATACAGTATTAG
AAGAAATAGATTTGCCAGGAAGATGGAAACCAAAAATCATAGGGGGAATT
GGAGGCTTTGTCAAAGTAAGAGAGTATGATCAARTACCCATAGAAATCTG
TGGAAAGAAAGTTATAGGTACAGTATTAGTAGGACCTACACCTGCCAACA
TAATTGGAAGAAATCTGATGACTCAGATGGGTTGCACTTTAAATTTT

The differences are underlined. After translation into amino acids and comparison, the result looks like this:

| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|----|----|----|----|----|----|----|----|----|----|----|----|----|----|----|----|----|----|----|----|----|----|----|----|
| P | Q | V | T | L | W | Q | R | P | L | V | T | I | K | I | G | G | Q | L | K | E | A | L | L | D | T | G | A | D | D | T | V | L |
|   | I |   |   |   |   |   |   |   |    | L  |    |    |    |    |    |    |    |    | R  |    |    |    |    |    |    |    |    |    |    |    |    |    |

| 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 |
|----|----|----|----|----|----|----|----|----|----|----|----|----|----|----|----|----|----|----|----|----|----|----|----|----|----|----|----|----|----|----|----|----|
| E | E | M | S | L | P | G | R | W | K | P | K | M | I | G | G | I | G | G | F | I | K | V | R | Q | Y | D | Q | I | L | L | E | I |
|   |   | I | D |   |   |   |   |   |   |   |   |   |   | L |   |   |   |   |   | V |   |   |   | E |   |   |   | V/I | P |   |   |   |

| 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 |
|----|----|----|----|----|----|----|----|----|----|----|----|----|----|----|----|----|----|----|----|----|----|----|----|----|----|----|----|----|----|----|----|----|
| C | G | H | K | A | G | T | V | L | V | G | P | T | P | V | N | I | I | G | R | N | L | L | T | Q | I | G | C | T | N | F |  |  |
|   |   |   | K | V |   |   |   |   |   |   |   |   |   |   | A |   |   |   |   |   |   |   | M |   |   | M |   |   |   |   |  |  |

The rows and figures with the dark grey background are the positions within the protease section. The letters on the medium grey background show the amino acids in the reference sequence. The llight grey background shows the sequence under test: empty spaces mean that the amino acid is the same as the one in the reference sequence, bold and boxed amino acids are mutations that are known or suspected to be associated with resistance to therapy or drugs.

In this case, mutations would be: 10l, 20R, 36l etc. where the number represents the position and the letter the amino acid that has mutated.

### *Virtual Phenotype calculation*

To calculate the Virtual Phenotype, the concept of 'similar sequences' needs to be explained.

To determine similarity between sequences, one cannot just match the nucleotides or amino acids, because they not always match completely. This is due to a number of undocumented mutations that can be found in any sequence and the fact that different combinations of nucleotides lead to the same amino acid.

To be able to compare, we define anchor points, or 'Hot-spots' as they are called.

For each drug, a number of hot-spots is defined and continuously updated.

Example:

| Drug A | Mutation A \| Mutation B \| Mutation C \| Mutation D |
|--------|------------------------------------------------------|
|  | Mutation E Mutation F |
|  | Mutation G & Mutation H |
|  | (Mutation I \| Mutation J) & (Mutation K \| Mutation L) |
|  | Mutation M Mutation N \| Mutation E \| Mutation F |
|  | (Mutation M \| Mutation N \| Mutation E \| Mutation F) & Mutation G |
|  | Mutation O & Mutation P |
|  | Mutation Q Mutation R \| Mutation F |
|  | Mutation E & Mutation Q & Mutation G |
|  | Mutation R |

In this example, there are 10 hot spot descriptions related to the drug in question.

To compare the sequences, a list of profiles (one profile per drug that is tested) is determined for every sequence. The profile is determined by keeping count of matching and non-matching hot spots per drug.

In the above example, if a sequence would match hot spot 2, 5, 6, 7 and 9, the sequence would have a profile for this drug equal to '0100111010'. Every new profile is stored inside the database.

Every hot-spot keeps count of the sequences that match the mutations it states. Using this information, the system is able to retrieve all the sequences that have exactly the same profile by doing an intersection of the sets that match and by subsequently subtracting the sets that don't match. In stead of using sets of sequences, the systems uses the corresponding sets of phenotypic data; this increases the performance of the system.

The next step is to retrieve the phenotypic results for those sequences. They vary between none and well over 20.000. On these phenotypic results, a few calculations are executed, e.g. mean or median fold resistances can be calculated:

1.

$$\sqrt{\frac{n\sum x^2 - \left(\sum x\right)^2}{n(n-1)}}$$

2. The log of the standard deviation of all the Fold Resistance values is calculated: Where n is the amount of phenotypic determinations and x contains the individual fold resistance values.

3. The mean of all the Fold Resistance values is calculated

4. The outliers are determined using a value of 3Ò; these are the Fold Resistance value that are greater than (mean + (3 x STD)) or smaller than (mean - (3 x STD))

5. The corrected mean Fold Resistance is calculated on all the data minus the outliers

This corrected value is reported and used to determine resistance together with the cut-off values corresponding to that drug. All the calculated values are stored together with the profiles they were calculated for.

**Example 2**

One example of an embodiment of the present invention can be described by the following steps:

1. The gag-RT-PR sequence is entered into a computer as a text string;
2. The computer program scans the sequence for all mutations, and 'lists' all those that are known or suspected to play a role in the development of drug resistance;
3. The mutations are then listed against each of the drugs for which they affect sensitivity;
4. For each drug, the computer program interrogates a genotype database for previous samples with the same or similar mutations or sequences, relating to that drug. Primary mutations, those initial mutations that have a discernable effect on drug resistance, are searched in the database individually first. Secondary mutations, those that have subtle effects on resistance or increase viral fitness, are searched in groups. Typically there will be several hundred records that match the pattern of mutations for each drug;
5. Every time a match is found, for example, a previous sample with the same pr similar pattern of AZT mutations, the computer program locates the phenotype for that sample in the Virco phenotype database and stores it (expressed as a change in $IC_{50}$)
6. Finally, again for each drug, the program calculates the mean change in $IC_{50}$ from all the examples it has found and summarizes the distribution of sensitivities as the percentage that were sensitive (resistance is unlikely), intermediate resistance is uncertain) or resistant (resistance is likely; and (resistance is uncertain) or resistant (resistance is likely); and
7. The program may then generate a final report that lists, for each drug in turn:

A) The drug names
B) The mutations found in the genotype that affect sensitivity to that drug
C) The number of genotypes in the Virco data base for which phenotype data is available
D) The proportion of these that were sensitive, intermediate or resistant to that drug
E) The mean sensitivity score — as a change in $IC_{50}$.

The invention also provides, in one embodiment, a method of assessing effectiveness of a therapy on a patient by determining whether the phenotype of a biological sample is in a therapeutically effect range. A therapeutically effective range takes into account, among other variables, the therapy or therapies being examined, individual patient characteristics such as a patient's pharmacokinetics, and resistance of the disease causing agent. One of skill in the art may calculate a therapeutically effective range by using, for example, published therapy effectiveness ranges and pharmacokinetic models. (*See e.g.,* European Patent Application No. 00/203200.1, filed on September 15, 2000

The invention also provides methods of optimizing therapy for a patient and designing therapy for a patient. In one embodiment, the skilled artisan may optimize and/or design a therapy by comparing the phenotypes determined using the methods of the invention and choosing the therapy or therapies that would be most effective for treating a patient.

Figure 1 represents a sample report produced using the present invention.

Studies have shown the present inventive method to be more than 90% accurate in predicting the actual phenotype using a current genotype and phenotype database. As more data is added to a database, the chances of finding large numbers of exact matches for the mutational pattern of an individual will increase and the level of accuracy can be even higher.

## Example 3

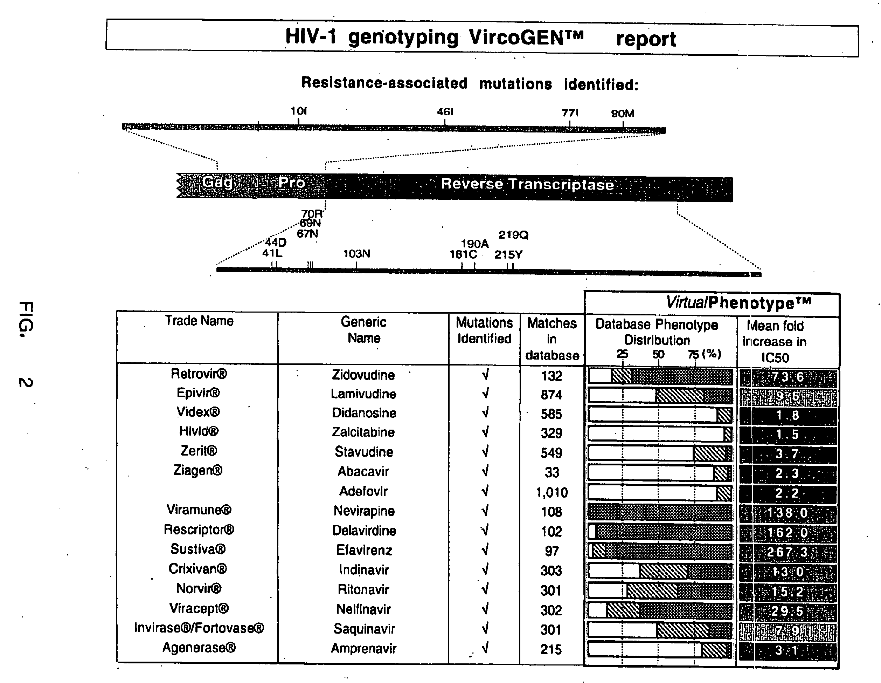

In the case shown in Figure 2, for example, the virus population is likely to respond to didanosine, zalcitabine, and stavudine (from the NRTIs), not AZT, 3TC and possibly not abacavir. A response is likely to any of the NNRTIs but the drug most likely to be effective is efavirenz. The patient's virus will very likely be resistant to the protease inhibitor nelfinavir and most iikeiy to be sensitive to amprenavir.

The distribution of the sensitivities of the phenotype matches can generally enable the physician, regardless of the disease studied, to select among alternative drugs that the system predicts will be effective to minimize the chances of resistance. With regard to HIV, for example, two protease inhibitors may have an identical score for the predicted change in $IC_{50}$, suggesting sensitivity, but one may have a wider spread of data, including some examples where there was resistance. The physician can then choose the drug with no evidence of resistance in the database.

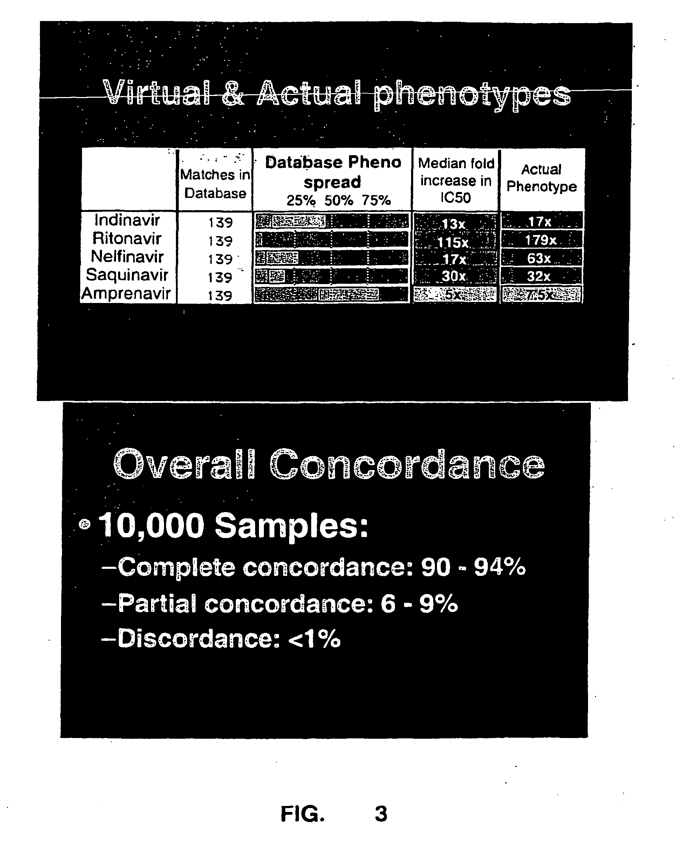

This mean sensitivity score is highly predictive of the actual phenotype and is therefore a reliable predictor of which drugs the patient will or will not respond to in the clinical setting. See Figure 3

## Example 4

In another embodiment, the present invention can be used with phenotypic resistance monitoring assays, such as known recombinant assays, in the clinical management of resistance developing diseases, including HIV and other viral infections, cancer, bacterial infections, and the like. A particularly useful resistance monitoring system is a recombinant assay known as the Antivirogram®. The Antivirogram® is a highly automated, high throughput, second generation, recombinant assay that can measure susceptibility, especially viral susceptibility, to all the available drugs, particularly antiretroviral drugs (reverse transcriptase inhibitors and protease inhibitors) at the same time. (Hertogs K, de Bethune MP, Miller V et al. Antimicrob Agents Chemother, 1998; 42(2):269-276).

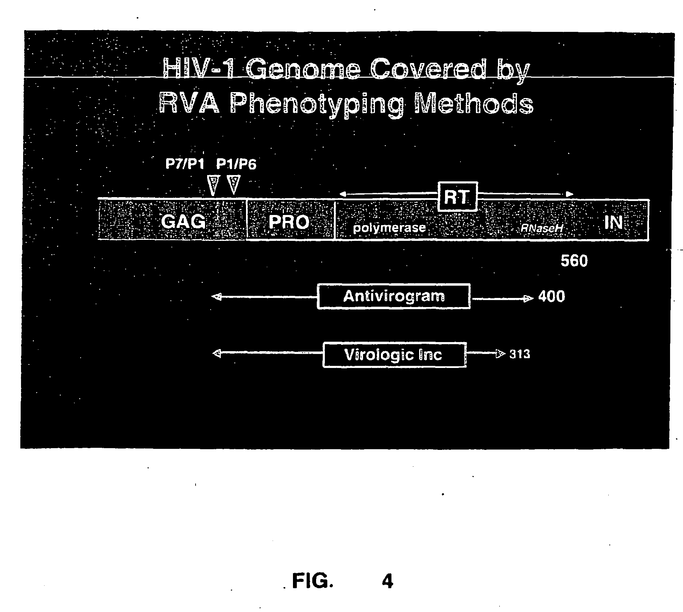

The whole process can be divided into three phases: molecular biology, transfection and susceptibility testing. The process is summarized below and in Figure 4.

**Molecular biology**

  ♦ Viral RNA fragments extracted from patient's blood sample
  ♦ Complementary DNA (cDNA) of the gag-PR-RT sequence, through to codon 400 formed via reverse transcription
  ♦ Gag-PT-RT sequence multiplied using two rounds of PCR
  ♦ Purification of the DNA fragments
  ♦ Creation of laboratory proviral clone with gag-PR-RT sequence deleted
  ♦ Insertion of the clone into bacterial plasmids for reproduction of large quantities

**Transfection**

This is the process by which viral genes are transferred to a cell.

  1. The gag-PR-RT sequences from the patient sample and the plasmid fragments are mixed with CD4+, MT4 cells.
  2. Electroporation takes place: the cells are subject to a short (milliseconds), but strong current in a cuvette producing transient openings in the cell membrane, through which both the gag-PR-RT DNA fragment and the plasmid fragment enter.
  3. In a relatively small proportion of the cells, both fragments will meet up and, probably supported by a cellular enzyme, recombine to form a complete HIV-1 genome that can now be converted into infectious virus particles.

4. The recombinant virus is then grown in this cell culture for approximately 8 days, until the cytopathogenic effect or CPE reaches a sufficient level.

5. The medium is then centrifuged to separate out the cells and the supernatant contains large quantities of recombinant virus — the virus stock harvest.

6. The virus is then titrated to achieve a known concentration.

**Susceptibility testing**

In this phase, it is determined if the different HIV-1 inhibitors are still capable of inhibiting replication of the recombinant viruses mentioned above.

1. Different concentrations of the antiviral agents are placed in the 384 microwells of a microtiter test plate. Several wells are used for each concentration and the mean results used to increase reliability.

2. A set dilution of the recombinant virus stock or wild type control virus is added to each microwell.

3. A set dilution of MT4 cells containing a fluorescent reporter gene system is also added to each microwell.

4. The plate is incubated for 3 days during which time the recombinant virus will replicate in the MT4 cells unless inhibited by the Antiviral drug. Replication triggers the reporter gene, which produces proteins which fluoresce.

5. The amount of viral replication at each concentration of drug is measured by computerized spectrophotometry, relative to the wild type virus controls.

6. The susceptibility of the virus to each drug is expressed as a fold change in $IC_{50}$ relative to wild type virus.

7. A report is prepared which provides these data for each drug with an increase in $IC_{50}$ of less than 4 classified as sensitive between 4 and 10 classified as intermediate and over 10 as resistant.

The whole process is highly automated and uses state of the art robotics to ensure consistency and high throughput.

Another assay exists that allows for simultaneous testing of susceptibility to reverse transcriptase inhibitors and protease inhibitors on a large scale: Virologics's 'Phenosense' assay (Petropoulos, CJ, Parkin NT, Limoli KL, et al. Antimicrob Agents Chemother, 2000; 44(4):920-928 .). The assay can be described as follows:

1. Viral RNA fragments are extracted from the patient's blood sample.

2. Complementary DNA (cDNA) of the gag-Pr-RT sequence to codon 300 is formed via reverse transcription.

3. Reverse transcriptase (RT) and protease (Pr)sequences are multiplied using PCR.

4. Sample RT-Pr sequences are ligated (joined) to provirus with the RT-Pr sequences deleted and an indicator gene, luciferase inserted in the deleted HIV-1 envelope gene.

5. These recombinant viral vectors, together with a plasmid carrying the envelope proteins of murine leukemia virus, are transfected into humans cells in the presence of varying concentrations of protease inhibitors.

6. Viral particles that are formed are harvested and allowed to infect target cells for a second time in the presence of various concentrations of RT inhibitors.

Susceptibility of the viral sequences to RT inhibitors and protease inhibitors is calculated by measurement of luciferase activity.

**Example 5**

It is desired to provide physicians and people living with diseases, in particular HIV/AIDS, with the most accurate, reliable and useful information about the individual person's disease to help them make the most informed decision about the optimal treatment strategy and to design treatment strategies. The methods of the present invention represented in one embodiment by the VircoGEN™ II, and the Antivirogram™, have a place in the clinical management of diseases, such as HIV/AIDS. The selection of which diagnostic test(s) to use and when is for the physician and his patient to make and depends on a number of different factors.

Recommendations for resistance testing are included in various treatment guidelines including those of the US Department of Health and Human Services and the International AIDS Society. They make no recommendations for which test to use other than the DHHS guidelines stating that the use of both tests is useful for people with complex treatment histories. The use of both phenotyping and genotyping is generally regarded as the most reliable approach to resistance testing.

Some clinical situations where resistance testing could be of value are listed below with some rational for the type of test to use.

The following table gives examples of clinical situations where resistance testing might be considered.

Table 1.

| Clinical situation | Assay/service | Rationale |
|---|---|---|
| Acute infection | VircoGEN II™ | At this point there is usually a high viral titer and any mutant virus that has been transmitted can be readily detected. |
| Initiation of therapy | VircoGEN II™ | At this point the patient is likely to have virus that is predominantly wild type or has a few mutations. It is, therefore, likely that the Virco database will have large numbers of matching records and that a *Virtual*/**Phenotype**™ will be highly reliable. |
| Sub-optimal response to potent combination therapy | VircoGEN II™ or BOTH | If the initial regimen was selected on the basis of genotypic information, then an Antivirogram™ should be run. If the initial selection was made without resistance information then a VircoGEN II may be sufficient. |
| Treatment failure | VircoGEN II™ | Again, when a patient's treatment regimen begins to fail, in most cases the number and complexity of the mutations are likely to be similar to samples run by Virco in the past, so the number of matches and the predictability of the Virtual/**PhenotypeT**™ will be high. |
| Treatment failure in patients with very complex treatment histories | BOTH | In this situation an Antivirogram™ is essential an running both tests would be best. Conducting bot tests means that the one can act as a check for the other. This combination will give how viruses with that pattern of mutations have 'behaved' in the pass and how this particular virus 'behaves' in the presence of drugs under controlled laborator conditions. |
| When new drugs are introduced | BOTH | In this situation there is likely to be a scarcity of information about the patterns of mutations involved in resistance - an Antivirogram™ would be essential and running both tests would be best. This would provide as much information as possible about the molecular basis of resistance to the new drug as well as informing clinical decision-making. |
| Few matches for the individual's genotype | Antivirogram™ | In a small minority of cases a genotype may reveal a novel pattern of mutations such that there are insufficient matches in the Virco database to produce a statistically reliable Virtua/**Phenotype**™. In these cases, an Antivirogram is recommended. |

### Example 6

**Sample source and susceptibility analysis.**

Plasma samples were obtained from patients and submitted to laboratories for routine assessment of drug susceptibility. These were collected mainly from the USA, Canada and Europe, although samples from South America, South East Asia and South Africa are also represented in the database. Due to the nature of collection of these samples, we were unable to obtain comprehensive therapy and clinical histories from the majority of the patients involved — although most were from different individual patients. Viral RNA was extracted from these samples and converted to cDNA by reverse transcription. Subsequently, a 1.7kb fragment of the HIV-1 genome that encompassed part of gag, the protease and the first 400 codons of RT was amplified by PCR[1]. These amplicons were directly sequenced by ABI automated sequencing and the drug susceptibility phenotype was determined for 14 ndividual antiretroviral drugs, using a recombinant virus assay. Text sequences were imported directly into the database, as were the $IC_{50}$ and fold resistance values for each drug.

**Database development and derivation of virtual phenotype.**

The genotype-phenotype database was developed in a RAD (Rapid Application Development) environment using Apple Macintosh. Programming was in "4th Dimension" (4D); a 32-bit, graphical, multi-threaded relational database. The database currently runs on a PowerMac G4, 400 MHz, 256 MB RAM. For the purposes of the analysis, the software assumed that the mixture of a wild type and mutant amino acid at a particular residue was mutant. A total of 108 individual, different amino acid changes were used in the search procedure (at a total of 56 unique positions). This was broken down into 39 changes in the protease and 69 in the RT (32 for the non-nucleoside RT inhibitors and 37 for the nucleoside analogues).

The following mutations, grouped by drug class, were included in the search engine. Protease inhibitors: 10F/I/R/V, 20I/M/R/T, 24I, 30N, 321, 33F/I/M/V, 361, 461/L, 47L, 48V, 50V, 54L/M/V, 71 TN, 73A/C/S, 771, 82A/F/S/T, 84A/V, 88D/S, 90M. Nucleoside analogues: 41 L, 44A/D, 62A, 65R, 67N, 69D/N, 69 insertion, 70R, 74V/I, 75A/I/M/T, 77L, 100I, 115F, 116Y, 118I, 151M, 181C, 184I/T/V, 208Y, 210W, 211K/Q, 215F/Y, 219E/N/Q, 333D/E. NNRTIs: 98G/S, 100I, 101E/I/P/Q, 103N/Q/R/S/T, 106A/I/L, 1081, 179D/E, 181C/I/N, 188C/H/L, 1891, 190A/E/S, 225H, 233V, 236L, 238T. At the time of the study, the database comprised ∼ 45,000 phenotyped and -35,000 genotyped samples, of which >15,000 had both a genotype and phenotype.

**DAP analysis of clinical samples.**

Viral load data of clinical samples from 191 patients who participated in the VIRA 3001 prospective HIV-1 phenotyping study were analysed according to the data analysis plan of the international resistance collaborative group. Complete phenotypic and genotypic data were available for these patients, who received a total of 635 antiretroviral drugs. The analysis parameter was virological failure at week 16, defined as plasma HIV-1 RNA above 400 copies/ml. Logistic regression was used to model this parameter. In the univariate models, the total genotypic sensitivity score (genotype analysis) or the phenotypic sensitivity score (real phenotype and virtual phenotype analysis) were the only factors in the model. Whereas, in the multivariate models, baseline HIV-1 plasma viral load and number of new drugs in the treatment regimen were added as extra covariates. To calculate the genotypic sensitivity score, particular mutations, or groups of mutations, were used to designate resistance or susceptibility to each antiretroviral drug in the regimen (these were pre-defined by the resistance collaborative group). Phenotypic sensitivity scores for both the actual phenotypes and virtual phenotypes were based on the fold change in $IC_{50}$ relative to a wild type, susceptible virus control. The total phenotypic score was defined as the number of susceptible drugs in the regimen.

**Derivation of the 'virtual phenotype'**

Firstly, the protease and reverse transcriptase (RT) regions of the HIV-1 genome were sequenced by standard methods. These regions code for the enzymes targeted by the current antiretroviral drugs and mutations here can confer drug resistance.

Mutations associated with resistance present in the sequence were identified and then software searched a relational genotype/phenotype database for archived samples with a similar mutation pattern for each drug (a mixture of wild type and mutant amino acid is treated as fully mutant). Because of the substantial size of the database, typically hundreds or thousands of matches were found. The software then retrieved the phenotypic data for each of the matching genotypes drug by drug, performed a logarithmic transformation and calculated a transformed mean fold-change in resistance.

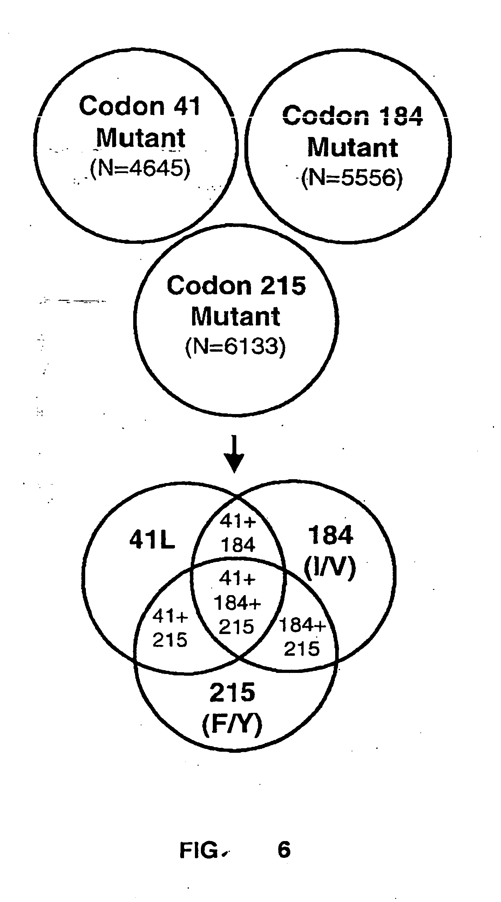

As with the actual phenotype on which it is based, this was expressed as a fold change in the 50% inhibitory concentration (IC$_{50}$) compared with a value of 1.0 for fully sensitive, wild type virus. Figure 6 shows diagrammatically how such a search was performed, using mutations that influence resistance to zidovudine (AZT) as an example. This illustration is for a virus that has any combination of the 41 L, 184V or I and 215Y or F mutations. A series of searches first find all samples that individually contain each of the mutations and then by an inclusion process, all samples containing the three illustrated mutations are identified.

Corresponding information from the database for these specific AZT resistance mutations is shown in Table 2. This illustrates examples of the first 13255 genotypically-matched samples found in the database for single and multiple mutations at HIV-1 RT codons 41, 184 and 215. A number of interesting characteristics are indicated in this Table. In particular, the phenotypic effect of a mutation depends upon the genetic context in which it occurs. In this simple example of only these three mutations, viruses with 41 L can have an average increase in resistance ranging from 1.3-fold to >27-fold. Thus, simple detection of the presence (or absence) of a given mutation can be uninformative or even misleading. Further, the effect of mutations is not simply additive - the modulation effects of the M184V or I mutations (decreasing AZT susceptibility) and/or the 41 L mutation (increasing AZT susceptibility) on viruses with the 215Y or F mutations can be discerned from Table X (range 6.2 to 27.7-fold). This analysis is considerably less sophisticated than the virtual phenotype system as it represents groups of samples where only the inclusion of three specific mutations has occurred, rather than the additional inclusion and exclusion of other mutations.

Table 2. Example of Method for Deriving AZT Virtual Phenotypes (using only three mutations).

| Codon 41 | Codon 184 | Codon 215 | Geometric Mean Phenotype | Average Phenotype (log) | Standard Deviation (log) | N |
|---|---|---|---|---|---|---|
| ANY | ANY | ANY | **3.9** | 0.59 | 0.78 | 13255 |
| WT | WT | WT | **1.3** | 0.12 | 0.38 | 4826 |
| WT | WT | F/Y | **13.4** | 1.13 | 0.73 | 695 |
| WT | V/I | WT | **1.3** | 0.10 | 0.47 | 2172 |
| WT | V/I | F/Y | **6.2** | 0.79 | 0.61 | 673 |
| L | WT | WT | **1.7** | 0.24 | 0.36 | 54 |
| L | WT | F/Y | **27.7** | 1.44 | 0.69 | 1783 |
| L | V/I | WT | **1.3** | 0.13 | 0.45 | 75 |
| L | V/I | F/Y | **15.2** | 1.18 | 0.69 | 2693 |

In the actual derivation of a Virtual Phenotype for AZT, a total of 18 mutations was examined in this fashion.

**Identification of genetic clusters with distinct phenotypes**

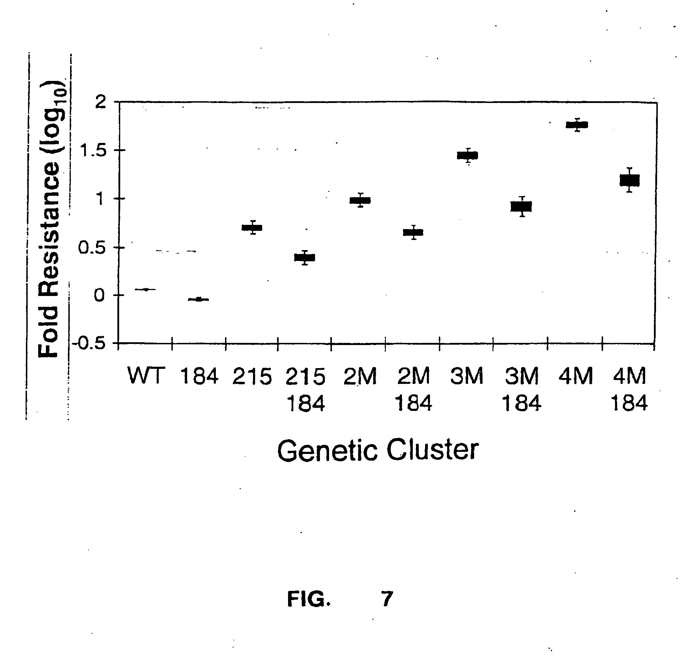

If the search process were functioning appropriately, a large series of phenotypically distinct genetic clusters should be identified. Each of these should have distinguishable phenotypes with only modest variability in susceptibility. This was evaluated by examining the genetic clusters formed by the combinations of AZT mutations described in Table 2. In addition to these mutations, clusters were identified that also contained additional AZT-resistance mutations (Fig 7). Searches of the database were performed using samples with specific AZT resistance mutations, with or without the 3TC resistance mutations, 184V or I. The numbers of samples in each genetic cluster were as follows: WT (wild type, susceptible), 3798; 184 (184V/I), 777; 215 (215Y/F), 175; 215 184 (215Y/F and 184V/I), 70; 2M (41 L and 215Y/F), 243; 2M 184 (41L, 215Y/F and 184V/I), 186; 3M (41 L, 210W and 215Y/F), 289; 3M 184 (41L, 210W, 215Y/F and 184V/I); 4M (41L, 67N, 210W and 215Y/F), 358; 4M 184 (41L, 67N, 210W, 215Y/F and 184V/I), 84.

This illustrates a number of important points regarding the database searches. Firstly, different genetic clusters have distinct susceptibility profiles (indicated by mean fold resistance values, together with the standard error and 95% confidence intervals). These values range from a slightly reduced level of susceptibility (virus harbouring the 184V mutation) to almost 100-fold increases, due to multiple mutations conferring AZT resistance. Secondly in each case, the inclusion of the 184V mutation together with AZT resistance mutations, caused a substantial reduction in the predicted magnitude of AZT resistance. The data clearly shows that the pattern recognition system can predict altered susceptibility due to interactions of mutations.

**Correlation between predicted and actual phenotype**

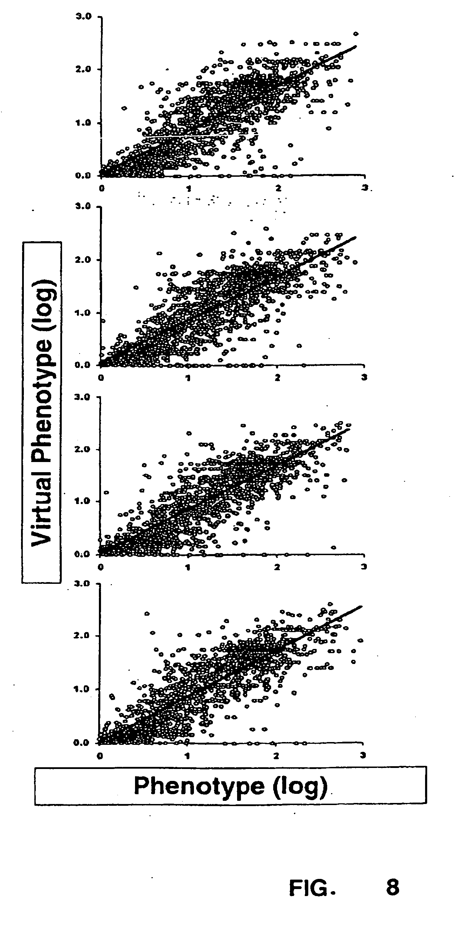

The virtual phenotype was validated in a number of ways. Firstly, between 2700 and 8700 genotypically wild type samples were tested for each drug. As anticipated, the predicted fold change was close to one for all drugs examined, with a range of 0.66 - 1.69 fold. Next, the quantitative relationship between the predicted phenotypes and actual phenotypes was investigated. 5000 clinically-derived samples from the USA were randomly selected from the resistance database from 1999 onwards and the phenotypic predictions obtained from the genotypic profiles for each drug were compared to actual phenotypes in 10 random subsets of 500 samples each. This resulted in approximately 70,000 determinations in total. Independent linear regression analyses were then performed on each of these data sets (four of these analyses are shown in Fig. 8). These showed a good correlation between the virtual phenotype (mean fold change in $IC_{50}$ value) and actual drug susceptibility phenotype, with an average slope of 0.83 (range 0.81-0.85), intercept of 0.05 (range 0.02 - 0.07) and average correlation coefficient of 0.87 (range 0.86 - 0.89) across the ten groups of 500 clinical samples.

**The virtual phenotype predicts clinical response**

The predictive value of the virtual phenotype was also tested. To address this, we performed a retrospective analysis of clinical and virological data from the clinical study, VIRA 3001. Cohen, C., et al., XIII International AIDS Conference. *Durban.* (2000). This is a recently completed prospective, randomized, clinical trial that demonstrated the positive effect of phenotypic drug resistance information on virological response in patients who had failed a PI-containing therapeutic regimen.

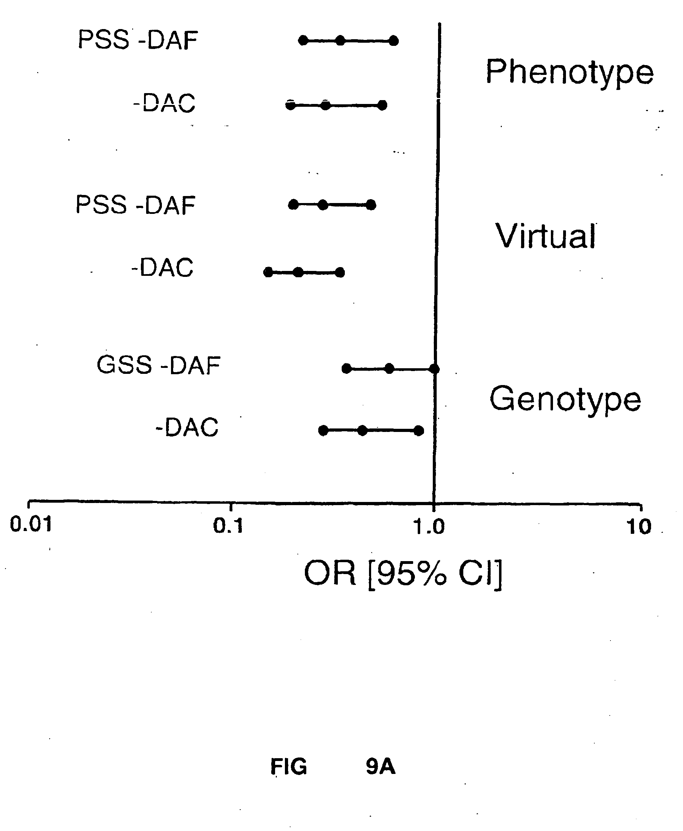

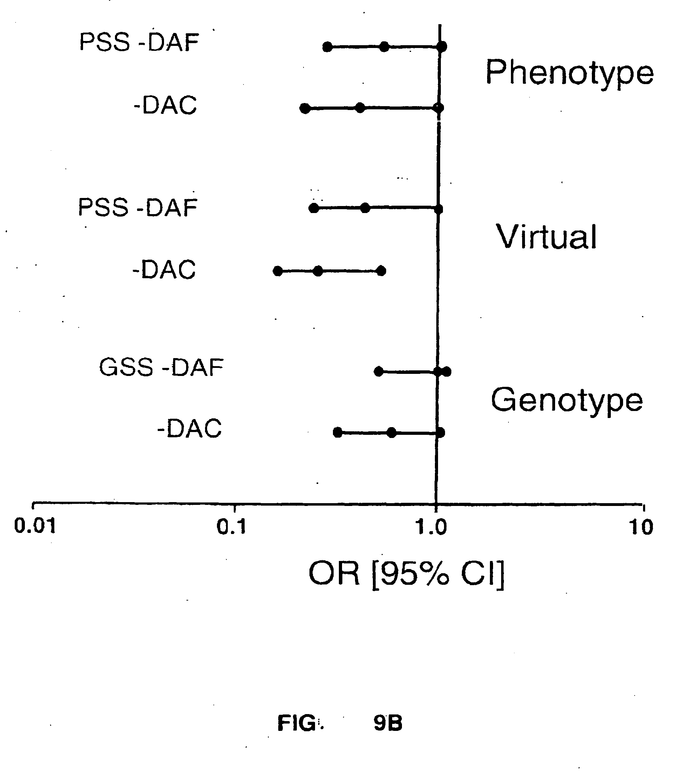

Samples from 191 patients in this study were re-analysed to test the relationship between the virtual phenotype (from genetic sequence) and virological outcome at 16 weeks. The predictive values of phenotype, virtual phenotype and genotype with 'rules-based' interpretation, were analysed according to a data analysis plan (DAP) used by the international resistance collaborative group to re-analyse clinical trials. DeGruttola V., et al., Antiviral Therapy 5, 41-48 (2000). This analysis system comprises univariate and multivariate statistical approaches and requires the use of a 'rules-based' mutation list for genotypic interpretation. The results of this analysis are shown in Fig. 9. Logistic regression was used to model the parameter of virological failure at week 16 (defined as plasma HIV-1 RNA above 400 copies/ml).Univariate (a) or multivariate (b) models were used for the drug susceptibility phenotype (phenotype), virtual phenotype (virtual) or genotype. The calculated phenotypic sensitivity score (PSS) or genotypic sensitivity score (GSS) were derived separately for a drop outs as censored (DAC) or drop outs as failures (DAF) analysis. Results of the regression analysis are shown on the Figure 9 as an odds ratio (OR) of failure to achieve a viral load reduction below 400 copies/ml, with the 95% confidence interval (CI).

In the univariate model, the genotype analysis (dropouts as censored, DAC) was a significant predictor of response with an odds ratio (OR) of 0.69 (CI=0.51-0.93), p=0.015 (Fig. 9a). However, the genotype was not a significant predictor of response in the multivariate model, OR=0.81 (CI=0.57-1.14), p=0.22 (Fig. 9b). In contrast, the virtual phenotype was highly significant in both models, also using the DAC analysis. With a 4-fold susceptibility cut-off for all drugs in the univariate model, the OR=0.38 (CI=0.25-0.6), p<0.0001 and in the multivariate model the OR=0.52 (cm.31-0.87), p=0.013. Using recently defined, drug-specific, biological cut offs, the predictive power of the virtual phenotype was even more significant. Larder, B. A. & Harrigan, P. R., Fifth International Congress on Drug Therapy in HIV Infection, Glasgow (2000). The OR in the univariate model was 0.39 (CI=0.26-0.58), p<0.0001, and in the multivariate model the OR=0.49 (CI=0.31-0.76), p=0.0014. The DAF (dropouts as failures) analyses showed consistent superiority for the predicted phenotype over genotype although the level of significance was correspondingly lower for all of the categories.

**Claims**

1.  A method of determining a virtual phenotype of a human immunodeficiency virus comprising:

    a) obtaining a genetic sequence of said virus
    b) identifying at least one mutation pattern-in said genetic sequence
    wherein said genetic sequence comprises at least one mutation, and
    wherein said at least one mutation or mutation pattern is associated with resistance to at least one therapy,
    c) searching a genotype database for genotype entries with a similar mutation pattern to at least one of the mutation patterns identified in the genetic sequence in b),
    d) correlating each genotype entry with a similar mutation pattern with a phenotype in a phenotype database, and,
    e) calculating the virtual fold resistance of said virus from all the database phenotypes identified.

2.  A diagnostic method for assessing the effectiveness of a patient's therapy comprising:

    a) providing a biological sample from a patient,

b) obtaining a genetic sequence from a human immunodeficiency virus in said biological sample,

c) identifying at least one mutation pattern in said genetic sequence wherein said genetic sequence comprises at least one mutation, and wherein said at least one mutation or mutation pattern is associated with resistance to at least one therapy currently being administered to the patient,

d) searching a genotype database for genotype entries with a similar mutation pattern to the at least one mutation pattern identified in the genetic sequence in c),

e) correlating said genotype entries with a similar mutation pattern with a phenotype in a phenotype database,

f) calculating the virtual fold resistance of said virus from all the database phenotypes identified,

g) obtaining a series of phenotypes by repeating steps b) through e) for each therapy currently being administered to the patient,

h) evaluating the effectiveness of the patient's therapy from the series of phenotypes.

3. A diagnostic method for optimizing therapy for a patient, comprising :

a) providing a biological sample from a patient,

b) obtaining a genetic sequence from a human immunodeficiency virus in said biological sample,

c) identifying at least one mutation pattern in said genetic sequence wherein said genetic sequence comprises at least one mutation, and wherein said at least one mutation or mutation pattern is associated with resistance to at least one therapy,

d) searching a genotype database for genotype entries with a similar mutation pattern to the at least one mutation pattern identified in the genetic sequence in c),

e) correlating said genotype entries with a similar mutation pattern with a phenotype in a phenotype database,

f) calculating the virtual fold resistance of said virus from all the database phenotypes identified

g) obtaining a series of phenotypes by repeating steps b) through e) for a group of therapies, and,

i) optimizing therapy for the patient from the series of phenotypes.

4. A diagnostic method for predicting resistance of a human immunodeficiency virus to therapy comprising:

a) providing a biological sample from a patient containing a human immunodeficiency virus,

b) obtaining a genetic sequence from said virus,

c) identifying at least one mutation pattern in said genetic sequence wherein said genetic sequence comprises at least one mutation, and wherein said at least one mutation or mutation pattern is associated with resistance to at least one therapy,

d) searching a genotype database for genotype entries with a similar mutation pattern to the at least one mutation pattern identified in the genetic sequence in c),

e) correlating said genotype entries with a similar mutation pattern with a phenotype in a phenotype database,

f) obtaining a series of phenotypes by repeating steps b) through e) for a group of therapies, and,

g) predicting resistance of the patient to therapy from the series of phenotypes.

5. The method of any of claims 1 to 4 wherein said virus is obtained from a biological sample chosen from a blood sample, a biopsy sample, a plasma sample, a saliva sample, a tissue sample, and a bodily fluid or mucous sample.

6. The method of claim 5, wherein the genetic sequence of HIV comprises the genetic sequence of the protease region of the HIV genome and/or the genetic sequence of the reverse transcriptase region of the HIV genome

7. The method of any of claims 1 to 6, wherein the mutation pattern comprises at least two mutations known or suspected to be associated with resistance to at least one therapy.

8. The method of any of claims 1 to 7, wherein the similar mutation pattern is identified by aligning the genetic sequence of a cell or a pathogen in the biological sample with the WT genetic sequence of said cell or pathogen.

9. The method of any of claims 1 to 8, wherein cluster searching is used to determine similar mutation patterns.

10. The method of any of claims 1 to 9, wherein a relational genotype/phenotype database is used for correlating the at least two genotype entries with a similar mutation pattern with a phenotype in said database.

11. The method of any of claims 1 to 10, wherein the phenotype of said biological sample is (expressed as) a mean fold-change in resistance towards at least one therapy, wherein said mean fold resistance is calculated from the database phenotype of the at least one genotype entry with a similar mutation pattern.

12. The method of any of claims 1 to 11, wherein the phenotype of the cell or pathogen in said biological sample is expressed as an $IC_{50}$.

13. A method of generating a report, wherein said report comprises the phenotype determined (predicted) using any of the methods of claims 1 to 12.

14. A method of determining a virtual phenotype of a human immunodeficiency virus comprising:

   a) obtaining a genetic sequence of said virus,
   b) identifying at least one mutation in said genetic sequence wherein said mutation is comprised within at least one mutation pattern,
   c) searching a genotype database for similar genotype entries comprising said mutation in said at least one mutation pattern,
   d) correlating said genotype entries with a phenotype in a phenotype database, and
   e) calculating the virtual fold resistance of said virus from all the database phenotypes identified.

15. A method according to claim 14 wherein said mutation pattern is associated with resistance to one therapy.

16. A method according to claim 14 or 15 wherein said mutation pattern comprises at least two mutations linked with a logical operator.

17. A method according to claims 14 - 16, wherein at least two mutation patterns are associated with resistance to one therapy.

18. A method according to claim 17, wherein said mutation patterns are linked with a logical operator defining a therapy profile.

19. A method according to claim 18, wherein said therapy profile is represented by a sequence.

20. A method according to claim 19, wherein said sequence is represented by a series of 1 and/or 0.

21. A method according to claim 20, wherein 1 represents the presence of a mutation pattern in the therapy profile and 0 the absence of a mutation pattern in the therapy profile.


**Patentansprüche**

1. Methode zur Bestimmung eines virtuellen Phänotyps eines menschlichen Immunschwächevirus, bei der man:

   a) eine genetische Sequenz des Virus gewinnt,
   b) wenigstens ein Mutationsmuster in der genetischen Sequenz identifiziert, wobei die genetische Sequenz wenigstens eine Mutation umfaßt und wobei die wenigstens eine Mutation bzw. das wenigstens eine Mutationsmuster mit Resistenz gegen wenigstens eine Therapie assoziiert ist,
   c) eine Genotypdatenbank auf Genotypeinträge mit einem zu wenigstens einem der in der genetischen Sequenz unter b) identifizierten Mutationsmuster ähnlichen Mutationsmuster durchsucht,
   d) die Genotypeinträge mit ähnlichem Mutationsmuster jeweils mit einem Phänotyp in einer Phänotypdatenbank korreliert und
   e) die virtuelle x-fache Resistenz des Virus aus allen in der Datenbank identifizierten Phänotypen errechnet.

2. Diagnostische Methode zur Beurteilung der Wirksamkeit einer Patiententherapie, wobei man bei der Methode:

   a) eine biologische Probe aus einem Patienten bereitstellt,
   b) eine genetische Sequenz aus einem menschlichen Immunschwächevirus in der biologischen Probe gewinnt,
   c) wenigstens ein Mutationsmuster in der genetischen Sequenz identifiziert, wobei die genetische Sequenz wenigstens eine Mutation umfaßt und wobei die wenigstens eine Mutation bzw. das wenigstens eine Mutationsmuster mit Resistenz gegen wenigstens eine gegenwärtig auf den Patienten angewandte Therapie assoziiert ist,

d) eine Genotypdatenbank auf Genotypeinträge mit einem zu dem in der genetischen Sequenz unter c) identifizierten wenigstens einen Mutationsmuster ähnlichen Mutationsmuster durchsucht,

e) die Genotypeinträge mit ähnlichem Mutationsmuster mit einem Phänotyp in einer Phänotypdatenbank korreliert,

f) die virtuelle x-fache Resistenz des Virus aus allen in der Datenbank identifizierten Phänotypen errechnet,

g) eine Reihe von Phänotypen erhält, indem man die Schritte b) bis e) für jede gegenwärtig auf den Patienten angewandte Therapie wiederholt,

h) die Wirksamkeit der Patiententherapie über die Reihe von Phänotypen bewertet.

3. Diagnostische Methode zur Optimierung der Therapie für einen Patienten, wobei man bei der Methode:

a) eine biologische Probe aus einem Patienten bereitstellt,

b) eine genetische Sequenz aus einem menschlichen Immunschwächevirus in der biologischen Probe gewinnt,

c) wenigstens ein Mutationsmuster in der genetischen Sequenz identifiziert, wobei die genetische Sequenz wenigstens eine Mutation umfaßt und wobei die wenigstens eine Mutation bzw. das wenigstens eine Mutationsmuster mit Resistenz gegen wenigstens eine Therapie assoziiert ist,

d) eine Genotypdatenbank auf Genotypeinträge mit einem zu dem in der genetischen Sequenz unter c) identifizierten wenigstens einen Mutationsmuster ähnlichen Mutationsmuster durchsucht,

e) die Genotypeinträge mit ähnlichem Mutationsmuster mit einem Phänotyp in einer Phänotypdatenbank korreliert,

f) die virtuelle x-fache Resistenz des Virus aus allen in der Datenbank identifizierten Phänotypen errechnet,

g) eine Reihe von Phänotypen erhält, indem man die Schritte b) bis e) für eine Gruppe von Therapien wiederholt und

h) die Therapie für den Patienten über die Reihe von Phänotypen optimiert.

4. Diagnostische Methode zur Vorhersage der Resistenz eines menschlichen Immunschwächevirus gegen Therapie, wobei man bei der Methode:

a) eine biologische Probe aus einem Patienten mit einem menschlichen Immunschwächevirus bereitstellt,

b) eine genetische Sequenz aus dem Virus gewinnt,

c) wenigstens ein Mutationsmuster in der genetischen Sequenz identifiziert, wobei die genetische Sequenz wenigstens eine Mutation umfaßt und wobei die wenigstens eine Mutation bzw. das wenigstens eine Mutationsmuster mit Resistenz gegen wenigstens eine Therapie assoziiert ist,

d) eine Genotypdatenbank auf Genotypeinträge mit einem zu dem in der genetischen Sequenz unter c) identifizierten wenigstens einen Mutationsmuster ähnlichen Mutationsmuster durchsucht,

e) die Genotypeinträge mit ähnlichem Mutationsmuster mit einem Phänotyp in einer Phänotypdatenbank korreliert,

f) eine Reihe von Phänotypen erhält, indem man die Schritte b) bis e) für eine Gruppe von Therapien wiederholt und

g) Resistenz des Patienten gegen Therapie über die Reihe von Phänotypen vorhersagt.

5. Methode nach einem der Ansprüche 1 bis 4, wobei man das Virus aus einer aus einer Blutprobe, einer Biopsieprobe, einer Plasmaprobe, einer Speichelprobe, einer Gewebeprobe sowie einer Körperflüssigkeits- oder Schleimprobe gewählten biologischen Probe gewinnt.

6. Methode nach Anspruch 5, wobei die genetische HIV-Sequenz die genetische Sequenz des Proteasebereichs des HIV-Genoms und/oder die genetische Sequenz des Reverse-Transkriptase-Bereichs des HIV-Genoms umfaßt.

7. Methode nach einem der Ansprüche 1 bis 6, wobei das Mutationsmuster wenigstens zwei Mutationen umfaßt, von denen bekannt ist oder vermutet wird, daß sie mit Resistenz gegen wenigstens eine Therapie assoziiert sind.

8. Methode nach einem der Ansprüche 1 bis 7, wobei man das ähnliche Mutationsmuster **dadurch** identifiziert, daß man die genetische Sequenz einer Zelle oder eines Krankheitserregers in der biologischen Probe der genetischen WT-Sequenz der Zelle bzw. des Krankheitserregers vergleichend gegenüberstellt.

9. Methode nach einem der Ansprüche 1 bis 8, wobei zur Bestimmung ähnlicher Mutationsmuster eine Cluster-Suche eingesetzt wird.

**10.** Methode nach einem der Ansprüche 1 bis 9, wobei eine relationale Genotyp-/Phänotypdatenbank für das Korrelieren der wenigstens zwei Genotypeinträge mit ähnlichem Mutationsmuster mit einem Phänotyp in der Datenbank verwendet wird.

**11.** Methode nach einem der Ansprüche 1 bis 10, wobei der Phänotyp der biologischen Probe eine mittlere x-fache Änderung der Resistenz gegenüber wenigstens einer Therapie darstellt (bzw. als eine solche Änderung ausgedrückt wird), wobei man die mittlere x-fache Resistenz aus dem Datenbankphänotyp des wenigstens einen Genotypeintrags mit ähnlichem Mutationsmuster errechnet.

**12.** Methode nach einem der Ansprüche 1 bis 11, wobei der Phänotyp der Zelle oder des Krankheitserregers in der biologischen Probe als $IC_{50}$-Wert ausgedrückt wird.

**13.** Methode zur Erstellung eines Berichts, wobei der Bericht den unter Verwendung einer der Methoden nach den Ansprüchen 1 bis 12 bestimmten (vorhergesagten) Phänotyp umfaßt.

**14.** Methode zur Bestimmung eines virtuellen Phänotyps eines menschlichen Immunschwächevirus, bei der man:

a) eine genetische Sequenz des Virus gewinnt,
b) wenigstens eine Mutation in der genetischen Sequenz identifiziert, wobei die Mutation in wenigstens einem Mutationsmuster enthalten ist,
c) eine Genotypdatenbank auf ähnliche Genotypeinträge durchsucht, die die Mutation in dem wenigstens einen Mutationsmuster enthalten,
d) die Genotypeinträge mit einem Phänotyp in einer Phänotypdatenbank korreliert und
e) die virtuelle x-fache Resistenz des Virus aus allen in der Datenbank identifizierten Phänotypen errechnet.

**15.** Methode nach Anspruch 14, wobei das Mutationsmuster mit Resistenz gegen eine Therapie assoziiert ist.

**16.** Methode nach Anspruch 14 oder 15, wobei das Mutationsmuster wenigstens zwei mit einem logischen Operator verknüpfte Mutationen umfaßt.

**17.** Methode nach den Ansprüchen 14-16, wobei wenigstens zwei Mutationsmuster mit Resistenz gegen eine Therapie assoziiert sind.

**18.** Methode nach Anspruch 17, wobei die Mutationsmuster mit einem ein Therapieprofil definierenden logischen Operator verknüpft sind.

**19.** Methode nach Anspruch 18, wobei das Therapieprofil durch eine Sequenz repräsentiert wird.

**20.** Methode nach Anspruch 19, wobei die Sequenz durch eine Folge von 1 und/oder 0 repräsentiert wird.

**21.** Methode nach Anspruch 20, wobei 1 das Vorhandensein eines Mutationsmusters im Therapieprofil und 0 die Abwesenheit eines Mutationsmusters im Therapieprofil repräsentiert.


**Revendications**

**1.** Procédé de détermination d'un phénotype virtuel d'un virus de l'immunodéficience humaine, comprenant les étapes consistant à :

a) obtenir une séquence génétique dudit virus,
b) identifier au moins un motif de mutation dans ladite séquence génétique, ladite séquence génétique comprenant au moins une mutation et ladite ou lesdites mutations ou ledit ou lesdits motifs de mutation étant associés à une résistance à au moins une thérapie,
c) chercher, dans une base de données de génotypes, des entrées de génotypes ayant un motif de mutation similaire à au moins l'un des motifs de mutation identifiés dans la séquence génétique en b),
d) corréler chaque entrée de génotype ayant un motif de mutation similaire à un phénotype dans une base de données de phénotypes, et
e) calculer le taux de résistance virtuelle dudit virus à partir de tous les phénotypes identifiés dans la base de données.

2. Procédé de diagnostic permettant d'évaluer l'efficacité de la thérapie d'un patient, comprenant les étapes consistant à :

a) obtenir un échantillon biologique d'un patient,
b) obtenir une séquence génétique d'un virus de l'immunodéficience humaine dans ledit échantillon biologique,
c) identifier au moins un motif de mutation dans ladite séquence génétique, ladite séquence génétique comprenant au moins une mutation et ladite ou lesdites mutations ou ledit ou lesdits motifs de mutation étant associés à une résistance à au moins une thérapie actuellement administrée au patient,
d) chercher, dans une base de données de génotypes, des entrées de génotypes ayant un motif de mutation similaire audit ou auxdits motifs de mutation identifiés dans la séquence génétique en c),
e) corréler lesdites entrées de génotypes ayant un motif de mutation similaire à un phénotype dans une base de données de phénotypes,
f) calculer le taux de résistance virtuelle dudit virus à partir de tous les phénotypes identifiés dans la base de données,
g) obtenir une série de phénotypes en répétant les étapes b) à e) pour chaque thérapie actuellement administrée au patient,
h) évaluer l'efficacité de la thérapie du patient d'après la série de phénotypes.

3. Procédé de diagnostic permettant d'optimiser la thérapie d'un patient, comprenant les étapes consistant à :

a) obtenir un échantillon biologique d'un patient,
b) obtenir une séquence génétique d'un virus de l'immunodéficience humaine dans ledit échantillon biologique,
c) identifier au moins un motif de mutation dans ladite séquence génétique, ladite séquence génétique comprenant au moins une mutation et ladite ou lesdites mutations ou ledit ou lesdits motifs de mutation étant associés à une résistance à au moins une thérapie,
d) chercher, dans une base de données de génotypes, des entrées de génotypes ayant un motif de mutation similaire audit ou auxdits motifs de mutation identifiés dans la séquence génétique en c),
e) corréler lesdites entrées de génotypes ayant un motif de mutation similaire à un phénotype dans une base de données de phénotypes,
f) calculer le taux de résistance virtuelle dudit virus à partir de tous les phénotypes identifiés dans la base de données,
g) obtenir une série de phénotypes en répétant les étapes b) à e) pour un groupe de thérapies, et
h) optimiser la thérapie du patient d'après la série de phénotypes.

4. Procédé de diagnostic permettant de prédire une résistance d'un virus de l'immunodéficience humaine à une thérapie, comprenant les étapes consistant à :

a) obtenir un échantillon biologique d'un patient contenant un virus de l'immunodéficience humaine,
b) obtenir une séquence génétique dudit virus,
c) identifier au moins un motif de mutation dans ladite séquence génétique, ladite séquence génétique comprenant au moins une mutation et ladite ou lesdites mutations ou ledit ou lesdits motifs de mutation étant associés à une résistance à au moins une thérapie,
d) chercher, dans une base de données de génotypes, des entrées de génotypes ayant un motif de mutation similaire audit ou auxdits motifs de mutation identifiés dans la séquence génétique en c),
e) corréler lesdites entrées de génotypes ayant un motif de mutation similaire à un phénotype dans une base de données de phénotypes,
f) obtenir une série de phénotypes en répétant les étapes b) à e) pour un groupe de thérapies, et
g) prédire la résistance du patient à la thérapie d'après la série de phénotypes.

5. Procédé selon l'une quelconque des revendications 1 à 4, selon lequel ledit virus est obtenu à partir d'un échantillon biologique choisi parmi un échantillon de sang, un échantillon de biopsie, un échantillon de plasma, un échantillon de salive, un échantillon de tissu et un échantillon de fluide corporel ou muqueux.

6. Procédé selon la revendication 5, selon lequel la séquence génétique de VIH comprend la séquence génétique de la région protéase du génome de VIH et/ou la séquence génétique de la région rétrotranscriptase du génome de VIH.

**7.** Procédé selon l'une quelconque des revendications 1 à 6, selon lequel le motif de mutation comprend au moins deux mutations que l'on sait être ou que l'on suspecte d'être associées à une résistance à au moins une thérapie.

**8.** Procédé selon l'une quelconque des revendications 1 à 7, selon lequel le motif de mutation similaire est identifié par alignement de la séquence génétique d'une cellule ou d'un pathogène dans l'échantillon biologique et de la séquence génétique de type sauvage de ladite cellule ou dudit pathogène.

**9.** Procédé selon l'une quelconque des revendications 1 à 8, selon lequel une recherche par groupes est utilisée pour déterminer des motifs de mutation similaires.

**10.** Procédé selon l'une quelconque des revendications 1 à 9, selon lequel une base de données relationnelle génotypes/phénotypes est utilisée pour corréler lesdites deux entrées de génotypes ou plus ayant un motif de mutation similaire à un phénotype dans ladite base de données.

**11.** Procédé selon l'une quelconque des revendications 1 à 10, selon lequel le phénotype dudit échantillon biologique est (exprimé sous forme d') un taux de changement de résistance moyen vis-à-vis d'au moins une thérapie, ledit taux de résistance moyen étant calculé d'après le phénotype de la base de données de ladite ou desdites entrées de génotypes ayant un motif de mutation similaire.

**12.** Procédé selon l'une quelconque des revendications 1 à 11, selon lequel le phénotype de la cellule ou du pathogène dans ledit échantillon biologique est exprimé sous forme d'une $CI_{50}$.

**13.** Procédé de génération d'un rapport, selon lequel ledit rapport comprend le phénotype déterminé (prédit) en utilisant l'un quelconque des procédés selon les revendications 1 à 12.

**14.** Procédé de détermination d'un phénotype virtuel d'un virus de l'immunodéficience humaine, comprenant les étapes consistant à :

    a) obtenir une séquence génétique dudit virus,
    b) identifier au moins une mutation dans ladite séquence génétique, ladite mutation étant comprise dans au moins un motif de mutation,
    c) chercher, dans une base de données de génotypes, des entrées de génotypes similaires comprenant ladite mutation dans ledit ou lesdits motifs de mutation,
    d) corréler lesdites entrées de génotypes à un phénotype dans une base de données de phénotypes, et
    e) calculer le taux de résistance virtuelle dudit virus à partir de tous les phénotypes identifiés dans la base de données.

**15.** Procédé selon la revendication 14, selon lequel ledit motif de mutation est associé à une résistance à une thérapie.

**16.** Procédé selon la revendication 14 ou 15, selon lequel ledit motif de mutation comprend au moins deux mutations liées par un opérateur logique.

**17.** Procédé selon les revendications 14 à 16, selon lequel au moins deux motifs de mutation sont associés à une résistance à une thérapie.

**18.** Procédé selon la revendication 17, selon lequel lesdits motifs de mutation sont liés par un opérateur logique définissant un profil thérapeutique.

**19.** Procédé selon la revendication 18, selon lequel ledit profil thérapeutique est représenté par une séquence.

**20.** Procédé selon la revendication 19, selon lequel ladite séquence est représentée par une série de 1 et/ou de 0.

**21.** Procédé selon la revendication 20, selon lequel 1 représente la présence d'un motif de mutation dans le profil thérapeutique et 0 l'absence d'un motif de mutation dans le profil thérapeutique.

FIG. 1

# HIV-1 genotyping VircoGEN™ report

## Resistance-associated mutations identified:

| Trade Name | Generic Name | Mutations Identified | Matches in database | VirtualPhenotype™ Database Phenotype Distribution 25 50 75 (%) | Mean fold increase in IC50 |
|---|---|---|---|---|---|
| Retrovir® | Zidovudine | √ | 132 | | 73.6 |
| Epivir® | Lamivudine | √ | 874 | | 9.6 |
| Videx® | Didanosine | √ | 585 | | 1.8 |
| Hivid® | Zalcitabine | √ | 329 | | 1.5 |
| Zerit® | Stavudine | √ | 549 | | 3.7 |
| Ziagen® | Abacavir | √ | 33 | | 2.3 |
| | Adefovir | √ | 1,010 | | 2.2 |
| Viramune® | Nevirapine | √ | 108 | | 138.0 |
| Rescriptor® | Delavirdine | √ | 102 | | 162.0 |
| Sustiva® | Efavirenz | √ | 97 | | 267.3 |
| Crixivan® | Indinavir | √ | 303 | | 13.0 |
| Norvir® | Ritonavir | √ | 301 | | 15.2 |
| Viracept® | Nelfinavir | √ | 302 | | 29.5 |
| Invirase®/Fortovase® | Saquinavir | √ | 301 | | 7.9 |
| Agenerase® | Amprenavir | √ | 215 | | 3.1 |

FIG. 2

| | Matches in Database | Database Pheno spread 25% 50% 75% | | Median fold increase in IC50 | Actual Phenotype |
|---|---|---|---|---|---|
| Indinavir | 139 | | | 13x | 17x |
| Ritonavir | 139 | | | 115x | 179x |
| Nelfinavir | 139 | | | 17x | 63x |
| Saquinavir | 139 | | | 30x | 32x |
| Amprenavir | 139 | | | 5x | 7.5x |

## Overall Concordance

- **10,000 Samples:**
  - Complete concordance: 90 - 94%
  - Partial concordance: 6 - 9%
  - Discordance: <1%

**FIG. 3**

FIG. 4

**FIG. 5**

FIG. 6

FIG.    7

EP 1 332 231 B1

FIG. 8

37

FIG     9A

FIG. 9B

100

Obtain a
genetic sequence
of a biological
sample

110

Search a relational
database for a
similar genetic
sequence

120

Obtain a database
phenotype for the
similar genetic
sequence

130

Calculate a
phenotype for the
biological sample

**FIG     10A**

Search genetic
sequence of
biological
sample for a
mutation related
to therapy "A"    202

Is that
mutation
present?    204

NO    The mutation
pattern receives a
"0"    206

YES

The mutation
pattern receives
a "1"    208

YES    Are there other
mutations
related to
therapy "A" ?    210

NO

Use database pheno-
type as phenotype of
biological sample    222

NO

Search a relational
database for all
database mutation
patterns with at
least one matching
mutation    212

Is there
more than
one match ?    220

YES    Calculate
phenotype of
biological
sample as mean
of all matching
database
phenotypes    224

Are any matching
database
mutation patterns
found ?    214

YES    Select matching
database mutation
pattern(s) with the
greatest number of
matching mutations    218

NO

Unable to
calculate virtual
phenotype    216

FIG    10B

12A

12N

```
┌─────────────┐      ┌─────────────┐
│ Health Care │      │ Health Care │
│  Provider   │──────│  Provider   │
└─────────────┘      └─────────────┘
```

30

Communication Channel

40

Phenotype
Determination

52

Public Database(s)

46

Local
Database(s)

FIG.    11

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9967427 A
- WO 0078996 A
- WO 9961658 A
- US 6087093 A
- WO 0073511 A
- US 09580491 A
- US 589167 A
- US 09580491 B
- WO 9727480 A
- EP 00203200 A


**Non-patent literature cited in the description**

- **LARDER et al.** *Science,* 1989, vol. 246, 1155-8
- **JAPOUR, A.J. ; MAYERS, T.L. ; JOHNSON, V.A. ; KURITZKES, D.R. ; BECKETT, L.A. ; ARDUINO, J.-M. ; LANE, J. ; BLACK, R.J. ; REICHELDERFER, P.S. ; D'AQUILA, R.T.** The RV-43 Study Group & The ACTG Virology Committee Resistance Working Group. *Antimicrob. Agents Chemother.,* 1993, vol. 37, 1095-1101
- **KELLAM, P. ; LARDER, B.A.** *Antimicrob. Agents Chemother.,* 1994, vol. 38, 23-30
- **LARDER BA ; KELLAM P ; KEMP, SD.** *AIDS,* 1991, vol. 5, 137-144
- **EASTMAN, P.S. ; URDEA, M. ; BESEMER, D. ; STEMPIEN, M. ; KOLBERG, J.** *J. Acquir. Immune Defic. Syndr. Human Retrovirol.,* 1995, vol. 9, 264-273
- **STUYVER, L. ; WYSEUR, A. ; ROMBOUT, A. ; LOUWAGIE, J. ; SCARCEZ, T. ; VERHOFSTEDE, C. ; RIMLAND, D. ; SCHINAZI, R. F. ; ROSSAU, R.** *Antimicrob. Agents Chemotherap.,* 1997, vol. 41, 284-291
- **D'AQUILA, R.T.** *Clin. Diagnost. Virol.,* 1995, vol. 3, 299-316
- *AIDS,* November 1998, vol. 12 (4), S11
- *Nucleic Acids Research,* 1998, 271-274
- **ALBERTS, B. ; BAY, D. ; LEWIS, J. ; RAFF, M. ; ROBERTS, K. ; WATSON, J.** The Molecular Biology of the Cell. Garland publishing, 1994
- **EDMAN P.** Sequence determination. *Mol. Biol. Biochem. Biophys.,* 1970, vol. 8, 211-255
- **YATES J.** Mass spectroscopy from genomes to proteomics. *Trends in genetics,* 2000, vol. 16, 5-8
- **SANGER F. ; NICHLER. ; COULSON A.** DNA sequencing with chain terminating inhibitors. *Proc. Nat. Acad. Sci.,* 1977, vol. 74, 5463-5467
- **GRABER J ; SMITH C. ; CANTOR C.** Differential sequencing with mass spectroscopy. *Genet. Anal.,* 1999, vol. 14, 215-219
- **FODOR S P ; RAVA R P ; HUANG X C ; PEASE A C ; HOLMES C P ; ADAMS C L.** Multiplexed biochemical assays with biological chips. *Nature,* 1993, vol. 364, 555-6
- **ROBERTSON et al.** *Nature Reviews,* 2000, vol. 1, 11-19
- **ESTELLER M. et al.** *New England Journal of Medicine,* 2000, vol. 343 (19), 1350-1354
- **SCHINAZI, R.F. ; LARDER, B.A. ; MELLORS, J.W.** *Int. Antiviral News.,* 1997, vol. 5, 129-142
- Bioinformatics: A practical guide to the analysis of genes and proteins. John, Wiley and Sons, 1998
- **G. SCHULER.** Sequence alignment and database searching
- **A. BAXEVANIS.** Aspects of multiple sequence alignment
- **M. HERSHKOVITZ ; D. LEIPE.** Phylogenetic analysis
- **SCHINAZI, R.F. ; LARDER, B.A. ; MELLORS, J.W.** *Int. Antiviral News,* 1997, vol. 5, 129-142
- **HERTOGS K ; DE BETHUNE MP ; MILLER V et al.** *Antimicrob Agents Chemother,* 1998, vol. 42 (2), 269-276
- **PETROPOULOS, CJ ; PARKIN NT ; LIMOLI KL et al.** *Antimicrob Agents Chemother,* 2000, vol. 44 (4), 920-928
- **COHEN, C. et al.** *XIII International AIDS Conference*
- **DEGRUTTOLA V. et al.** *Antiviral Therapy,* 2000, vol. 5, 41-48
- **LARDER, B. A. ; HARRIGAN, P. R.** Fifth International Congress on Drug Therapy in HIV Infection. Glasgow, 2000